# EUROPEAN PATENT SPECIFICATION

(11) **EP 3 806 734 B1**
(45) Date of publication and mention of the grant of the patent: **20.03.2024**
(21) Application number: 19734130.8
(22) Date of filing: 25.05.2019
(51) Int. Cl.: A61B 5/053, A61B 5/145, A61B 5/1468, A61B 5/00

(54) **EVALUATION OF AN AMOUNT OF A SUBSTANCE CONTAINED WITHIN CIRCULATING BLOOD**
BEURTEILUNG EINER MENGE EINER SUBSTANZ IM BLUTKREISLAUF
ÉVALUATION D'UNE QUANTITÉ D'UNE SUBSTANCE CONTENUE DANS LE SANG CIRCULANT

(30) Priority: 31.05.2018 GB 201808857; 27.04.2019 GB 201905972; 04.05.2019 GB 201906386
(43) Date of publication of application: 21.04.2021
(73) Proprietor: Zedsen Limited, London W4 5YA (GB)
(72) Inventor: MAMIGONIANS, Hrand Mami, London W5 3EB (GB); KANAKIS, Anastasios, Cambridge CB5 8PB (GB); MAMIGONIANS, Armen, London W5 1AW (GB); POLEC, Daniel loan, London W11 1WL (GB); SIVASAILAM, Vivekram, West Drayton UB7 9FF (GB); SULAIMALEBBE, Aslam, Cardiff CF24 3GN (GB)
(74) Representative: Atkinson & Company Intellectual Property Limited
(86) International application number: PCT/GB2019/000074
(87) International publication number: WO 2019/229407

(56) References cited:
- WO-A1-2017/046710
- WO-A2-2009/136311
- US-A1- 2009 312 615

## Description

The present invention relates to an apparatus for the non-invasive evaluation of an amount of a substance contained within blood circulating within human body tissue. The present invention also relates to a method of determining an amount of a substance contained within blood circulating within human body tissue.

It is known to examine objects using electric fields, as described in US 8,994,383 assigned to the present applicant. However, problems arise when examining objects that are not homogeneous, such as biological tissues.

It is also known to adjust the penetration of electric fields by selecting different combinations of transmitter electrode and receiver electrode, with different separation distances. Thus, as the distance between a transmitter electrode and a receiver electrode increases, a greater degree of penetration is achieved.

A further problem arises in terms of evaluating an amount of a substance (such as glucose) contained within blood circulating within human body tissue. In particular, it is necessary for a subject to establish skin contact with insulated electrodes positioned on a dielectric substrate. Experiment has shown that fingers provide good candidates for making evaluations of this type, given the relatively high concentration of capillaries close to the skin. However, experiments also suggest that measurements may be influenced by changes in temperature, humidity and applied pressure. In particular, it is necessary for a sufficient level of pressure to be applied in order to achieve satisfactory results via the capacitive coupling of displaced electrodes.

According to a first aspect of the present invention, there is provided a method of determining an amount of a substance contained within blood circulating within human body tissue, characterised by the steps of: locating an application region of an apparatus to make skin contact at the position of said tissue, wherein said apparatus includes a plurality of electrodes arranged on a dielectric substrate to present an active surface at said application region; deriving force data from a detector; comparing said force data, representing applied force/pressure of said skin contact upon said application region, against a first predetermined level; and in response to said comparing step, either: performing capacitive-coupling procedures that capacitively couple selected pairs of said electrodes by producing electric fields that penetrate said tissue to produce monitored output data, if sufficient force has been applied; or inhibiting said capacitive-coupling procedures if a sufficient force has not been applied, wherein the amount of said substance is evaluated from said monitored output data when said monitored output data is produced; characterised in that said step of performing capacitive-coupling procedures comprises the steps of: selecting a first set of n electrodes from said plurality of substantially parallel electrodes; for each of said electrodes in said first set: selecting a second set of m electrodes from said plurality of substantially parallel electrodes, wherein the electrodes in said second set are the nearest neighbouring electrodes to said each electrode, generating an energisation pulse for application to said each electrode as a transmitter electrode, and monitoring output signals from each of said electrodes in said second set as a receiver electrode, wherein a peak value of an output signal is indicative of permittivity and a decay rate of an output signal is indicative of conductivity, such that during each energisation operation, an energised transmitter electrode and a monitored receiver electrode define a capacitively-coupled electrode pair; such that a plurality of capacitively coupled electrode pairs are established, in which each electrode in said first set of electrodes is capacitively coupled with electrodes in a second set, and the number m represents a degree of layering.

According to a second aspect of the present invention, there is provided an apparatus for the non-invasive evaluation of an amount of a substance contained within blood circulating within human body tissue, comprising: an application region arranged to make skin contact; a plurality of substantially parallel electrodes arranged on a dielectric substrate, thereby presenting an active surface at the position of said application region; a detector configured to produce force data representing an applied force or pressure upon said active surface; and a processor, wherein said processor configured to: compare said force data against a first predetermined level; perform capacitive-coupling procedures that capacitively couple selected pairs of said electrodes by producing electric fields that penetrate said tissue to produce monitored output data, if said force data is higher than said first predetermined level; and inhibit said capacitive-coupling procedures if said force data is not above said first predetermined level; characterised in that said processor is configured to perform capacitive-coupling procedures by carrying out the steps of: selecting a first set of n electrodes from said plurality of substantially parallel electrodes; and for each of said electrodes in said first set: selecting a second set of m electrodes from said plurality of substantially parallel electrodes, wherein the electrodes in said second set are the nearest neighbouring electrodes to said each electrode, generating an energisation pulse for application to said each electrode as a transmitter electrode, and monitoring output signals from each of said electrodes in said second set as a receiver electrode, wherein a peak value of an output signal is indicative of permittivity and a decay rate of an output signal is indicative of conductivity, such that during each energisation operation, an energised transmitter electrode and a monitored receiver electrode define a capacitively-coupled electrode pair; such that a plurality of capacitively coupled electrode pairs are established, in which each electrode in said first set of electrodes is capacitively coupled with electrodes in a second set, and the number m represents a degree of layering.

In an embodiment, the apparatus includes a display device configured to indicate that an increase in applied force/pressure is required if the capacitive coupling procedures have been inhibited.

In an embodiment, the processor is also configured to inhibit the capacitive coupling procedures if the force data is above a second predetermined level. The apparatus may also include a display device configured to indicate that a reduction in applied force/pressure is required.

In an embodiment, the processor is also configured to store force data when the capacitive coupling procedures are performed.

Embodiments of the invention will be described, by way of example only, with reference to the accompanying drawings, of which:
Figure 1 shows a pressure-compensating non-invasive blood-containing substance measuring apparatus;
Figure 2 shows a main circuit board of the apparatus of Figure 1;
Figure 3 shows the underside of the main circuit board identified in Figure 2;
Figure 4 shows a cross section of the apparatus of Figure 1;
Figure 5 details a force sensor of the type shown in Figure 4;
Figure 6 shows a schematic representation of the apparatus shown in Figure 1;
Figure 7 shows a schematic representation of an energising circuit, of the type identified in Figure 6;
Figure 8 shows an example of the multiplexing environment identified in Figure 6;
Figure 9 illustrates two mutually orthogonal electrode groups;
Figure 10 shows an example of the analogue processing circuit identified in Figure 6;
Figure 11 shows procedures performed by the processor identified in Figure 6;
Figure 12 shows an example of a scan cycle;
Figure 13 illustrates a message inviting a subject to deploy a finger;
Figure 14 shows examples of applied force during an evaluation period;
Figure 15 illustrates a second message to a subject confirming that scanning operations are being performed;
Figure 16 illustrates a third message to a subject inviting them to press harder;
Figure 17 illustrates a fourth message to a subject inviting them to press less hard;
Figure 18 illustrates an output data block;
Figure 19 illustrates operations performed by a machine learning system;
Figure 20 shows a pattern of electric fields generated from a plurality of electrodes by an embodiment;
Figure 21 shows the relationship between an energising input pulse and a monitored output signal;
Figure 22 illustrates an embodiment for achieving the pattern identified in Figure 20, in which a first electrode is energised;
Figure 23 shows a common electrode data set produced from the coupling operations shown in Figure 22;
Figure 24 illustrates the energisation of a second electrode;
Figure 25 illustrates a common electrode data set produced from the coupling operations illustrated in Figure 24;
Figure 26 continues to illustrate a process of sequentially selecting adjacent electrodes as electrodes in common;
Figure 27 illustrates data produced from the energisation of electrode 14;
Figure 28 illustrates the start of reverse layering from electrode 15;
Figure 29 shows a common electrode data set by reverse layering;
Figure 30 shows a layering data set;

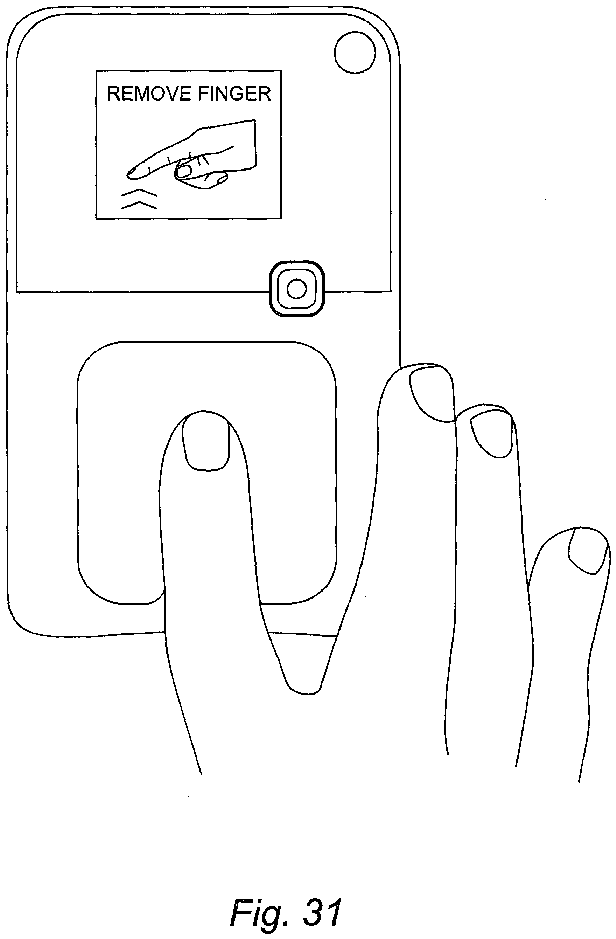

Figure 31 shows a third stage of operation, inviting the finger to be removed; and

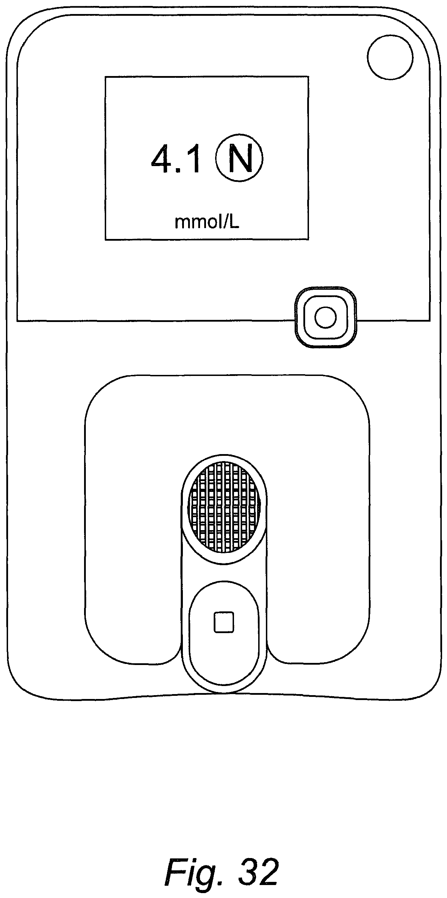

Figure 32 shows a fourth stage of operation, presenting an indication of glucose level.

### Figure 1

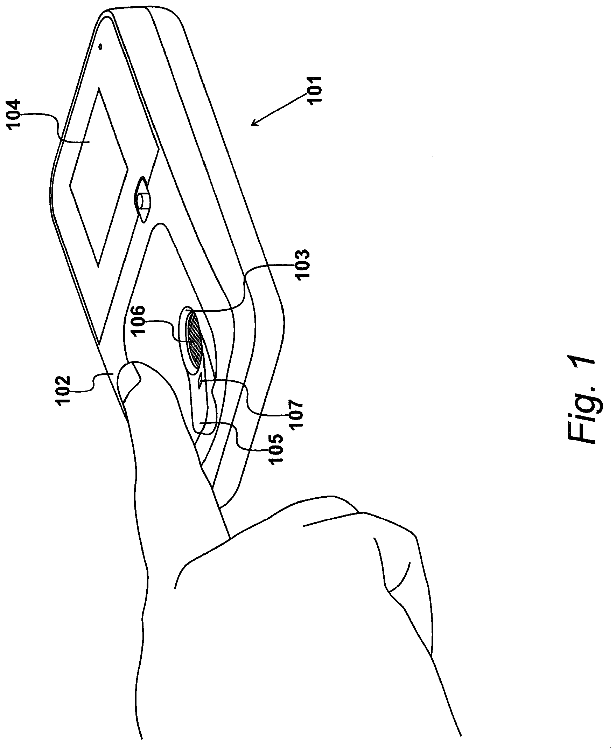

An apparatus **101** for the non-invasive evaluation of an amount of a substance contained within human body tissue is shown in Figure **1**. The apparatus has a plastic housing **102**, with a membrane-exposing orifice **103**, presenting an application region arranged to make skin contact. In this embodiment, contact is made against the skin of a finger, but in other embodiments, contact can be made with other suitable areas of the body, such as a wrist.

The housing also includes a visual display orifice **104** which, in this embodiment, is covered by a transparent cover, thereby allowing a visual display unit, supported by a main circuit board, to be seen during the operation of the apparatus. In this embodiment, the visual display unit is a liquid crystal display but other types of display can be deployed. In alternative embodiments, the display may take the form of devices configured to emit light of various colours. Alternatively, a display may be presented to a subject (the person being tested) or to an operative (such as a clinician) via an alternative device, such as a wireless connected mobile device.

A guide portion **105** guides a subject's finger into position, to contact with an electrode supporting membrane **106.** The guide portion **104** also includes a temperature senor **107.** An additional temperature sensor and a humidity sensor may also be included within the housing. In this way, each data set produced during a scanning operation may include temperature data and humidity data in addition to data representing a degree of applied force or pressure.

In this embodiment, a device measures applied force thereby allowing a processor to compare force data against a predetermined level. A minimum level of pressure is required to ensure that a reliable contact is made between the subject's finger and the electrode supporting membrane **106.** Thus, testing is inhibited if the force data is not above this predetermined level.

The electrodes are coated with a thin layer of an insulating material, such that an applied finger does not make electrical contact with the electrodes but does capacitively engage with the electrodes; such that it is possible for electric fields to enter the fingertip without an airgap being present.

### Figure 2

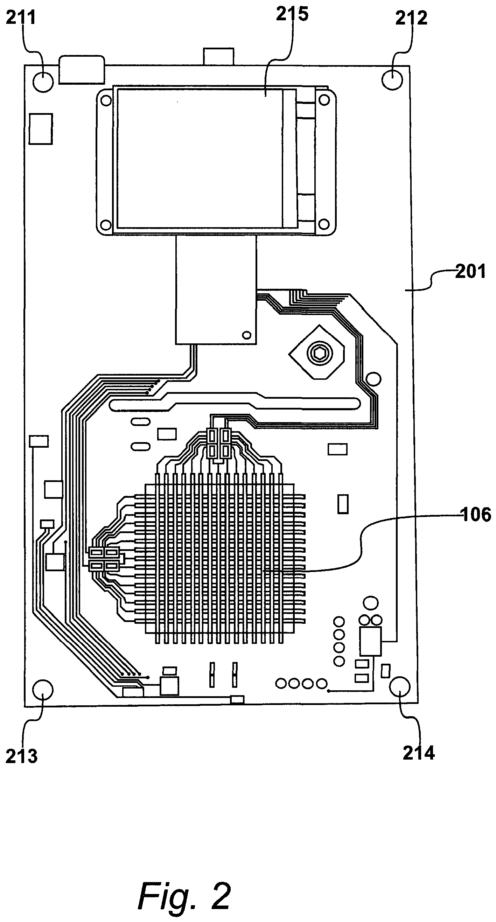

A main circuit board **201** is shown in Figure 2, upon which the electrode supporting membrane **106** is itself supported above a first orifice. In this way, when pressure is applied to the membrane **106**, a limited degree of movement is possible, resulting in force being applied to a force sensor.

A plurality of electrically insulated substantially parallel electrodes are mounted on the dielectric membrane **106** and the main circuit board **201** provides electrical connections to these electrodes. In this embodiment, in addition to a first group of substantially parallel electrodes mounted on the top surface of the dielectric membrane, a second group of substantially parallel electrodes are mounted on the underside of the dielectric membrane **106.**

The orientation of the second group of electrodes is offset with respect to the orientation of the first group of electrodes. In this embodiment, the first group of electrodes are mutually orthogonal to the second group of electrodes. In this way, it is possible for a first layering operation to be performed using the first group, followed by a second layering operation being performed using the second group. Conventional position detection is also possible by sequentially energising electrodes of one of these groups while monitoring selected electrodes of the other group.

Circuit board **201** is secured to the housing **102** at a first securing location **211**, a second securing location **212**, a third securing location **213** and a fourth securing location **214.** A visual display unit **215** is attached to the main circuit board **201**.

### Figure 3

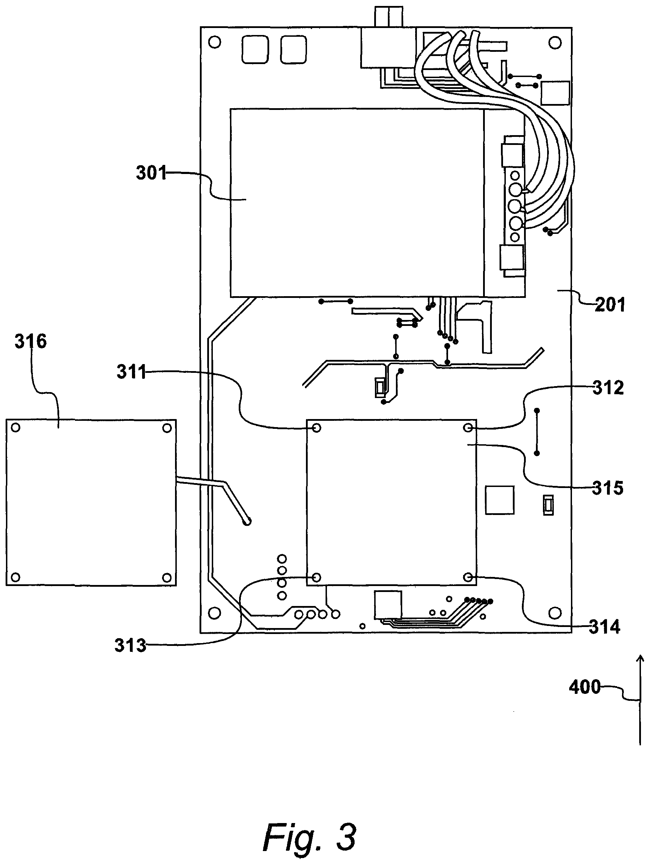

The underside of the main circuit board **201** is shown in Figure 3. A rechargeable battery **301** provides electrical power for components within the apparatus. Plural fixing elements, consisting of a first rod **311**, a second rod **312**, a third rod **313** and a fourth rod **314** are secured to the main circuit board **201.** In an embodiment, these fixing elements (rods) are secured by being soldered to circuit board **201.** In an embodiment, a plastic support **315** is located on rods **311** to **314** to support the dielectric membrane **106.** In an embodiment, the plastic support is derived from an acetyl material, selected such that electrical properties of this material do not change with respect to changes in temperature and humidity experienced within the operational environment.

Following the application of support **315**, an intermediate board **316** is deployed over the rods **311** to **314**, such that it is guided but not restrained by these fixing elements. In this way, board **316** is allowed to move and as such applies force onto the force sensor. In an embodiment, the intermediate board **316** includes an electrically conductive ground plane to provide electrical shielding to the lower side of the membrane **106.**

After deploying the intermediate board **316**, a bottom circuit board is located on the fixing elements **311** to **314** and thereafter secured to the fixing elements. Thus, the plural fixing elements secure the bottom circuit board to the top circuit board, such that the bottom circuit board does not move with respect to the main circuit board and the bottom circuit board does not contact the housing **102** directly.

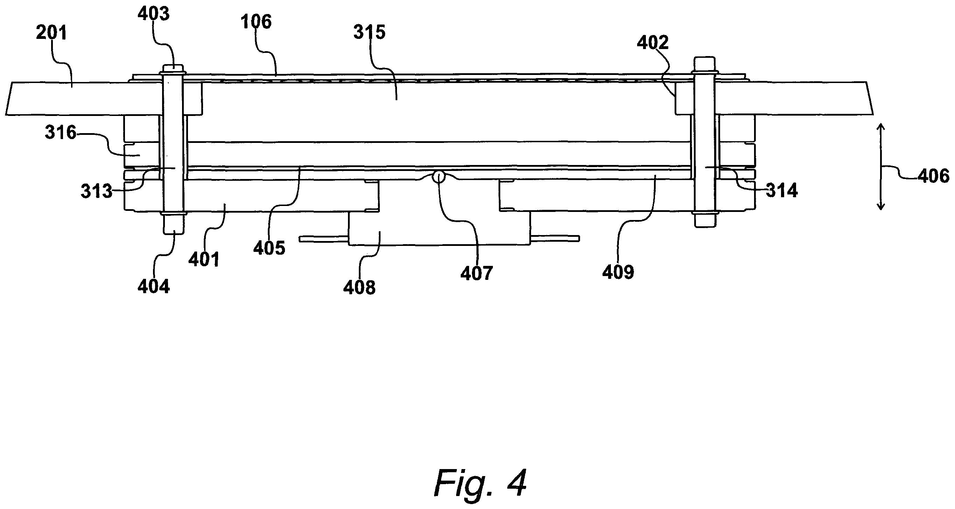

A cross-sectional view of the apparatus, looking in the direction of arrow **400**, is shown in Figure 4.

### Figure 4

Metal rod **313** and metal rod **314** are shown in the cross section of Figure 4. These, along with the other two fixing rods, secure a bottom circuit board **401** to the top circuit board **201.** The top circuit board **201** includes a first orifice **402** and the dielectric membrane **106** is supported over this orifice. In an embodiment, the membrane has a thickness of typically 0.1 millimetres and the main circuit board **201** has a typical thickness of 1.6 millimetres. Each of the metal rods, including metal rod **313**, has an upper end **403** and a lower end **404** such that, in an embodiment, the upper ends **403** are soldered to the main circuit board **201** and the lower ends **404** are soldered to the bottom circuit board **401.**

The acetyl support **315** is shown in Figure 4, along with the intermediate board **316** with a ground plane **405.** Support **315** and board **316** are guided by the fixing elements **311** to **314** but are not restrained by these fixing elements, such that they are free to move in a vertical direction, as indicated by arrow **406.** Relatively little movement may occur, typically up to a maximum of ten micrometres but it is unlikely that this would be perceived by a subject. Movement is restrained by a metal ball **407** extending from a force sensor **408**, wherein the metal ball **407** is in contact with the ground plane **405** attached intermediate board **316.**

In this embodiment, the force sensor **408** is received within an orifice provided within the bottom circuit board **401**, with the metal ball **407** extending above the plane of the bottom circuit board **401.** Thus, in this way, an extending portion of the force sensor extends above a top surface of the bottom circuit board.

In an embodiment, the extending portion is surrounded by an elastomeric material **409.** In an embodiment, the elastomeric material is a silicone rubber with a Shore durometer (type A) of less than forty. Thus, when flexing occurs, due to applied pressure, the elastomeric material **409** compresses. Thereafter, when force is removed, the elastomeric material will expand back to its original position, thereby ensuring that the apparatus is returned to a fully operational state.

Thus, in an embodiment, a subassembly is formed consisting of the bottom circuit board **401** and an inserted force sensor **408** with an extending portion surrounded by the elastomeric material **409.** This subassembly is then located over the fixing elements and soldered into position, as previously described.

### Figure 5

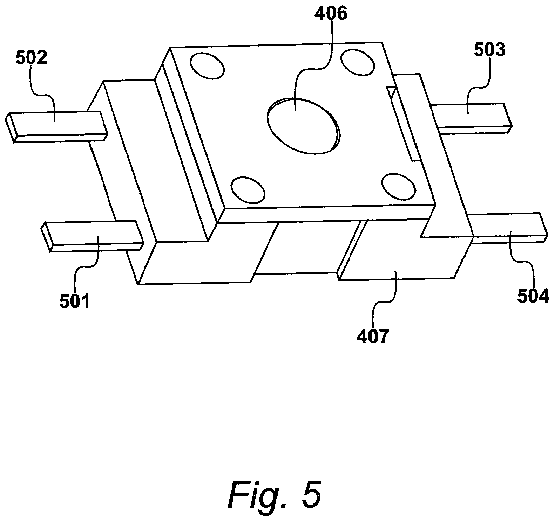

An example of force sensor **405** is illustrated in Figure 5, that may be an FSS low profile force sensor produced by Honeywell Corporation, Illinois USA. As illustrated in Figure 5, the metal ball (of stainless steel) is located at the centre of the sensor. Internally, the sensor uses a piezo-resistive micromachined silicone sensing element. The sensor is configured such that its resistance will increase when the sensing element flexes under an applied force. The stainless-steel ball **406** concentrates this force directly onto the silicon-sensing element. Thus, resistance changes in proportion to the amount of force being applied.

The device presents a first pin **501**, a second pin **502**, a third pin **503** and a fourth pin **504.** Up to a maximum of twelve volts is applied across the first pin **501** and the third pin **503.** Sensor output is then measured as a differential voltage across the second pin **502** and the fourth pin **504.** Other types of sensor may be deployed, such as those directly measuring pressure or strain.

### Figure 6

A schematic representation of an examination apparatus embodying the present invention is shown in Figure 4. A multiplexing environment **601** includes a dielectric membrane supporting at least one group of parallel electrodes. Environment **601** also includes a de-multiplexer for de-multiplexing energising input pulses, along with a multiplexer for combining selected output signals.

A processor **602**, implemented as a microcontroller, addresses the de-multiplexer and the multiplexer to ensure that the same electrode cannot be both energised as a transmitter and monitored as a receiver during the same coupling operation. An energising circuit **603** is energised by a power supply **604** that may in turn receive power from an external source via a power input connector **605.** A voltage-control line **606** from the processor **602** to the energising circuit **603**, allows processor **602** to control the voltage (and hence the energy) of the energising signals supplied to the multiplexing environment via a strobing line **607.** The timing of each energising signal is controlled by the processor **602** via a trigger-signal line **608.**

An output from the multiplexing environment **601** is supplied to an analogue-processing circuit **609** over a first analogue line **610.** A conditioning operation is performed by the analogue-processing circuit **609**, allowing analogue output signals to be supplied to the processor **602** via a second analogue line **611.** The processor **602** also communicates with a two-way-data-communication circuit **612** to facilitate the connection of a data-communication cable. In an alternative embodiment, communication with external devices is achieved using a wireless protocol.

During scanning operations, the processor **602** supplies addresses over address buses **614** to the multiplexing environment **601**, to define a pair of capacitively coupled electrodes. An energisation operation is performed by applying an energising voltage to strobing line **607**, monitoring a resulting output signal and sampling the output signal multiple times to capture data indicative of a peak value and a rate of decay.

### Figure 7

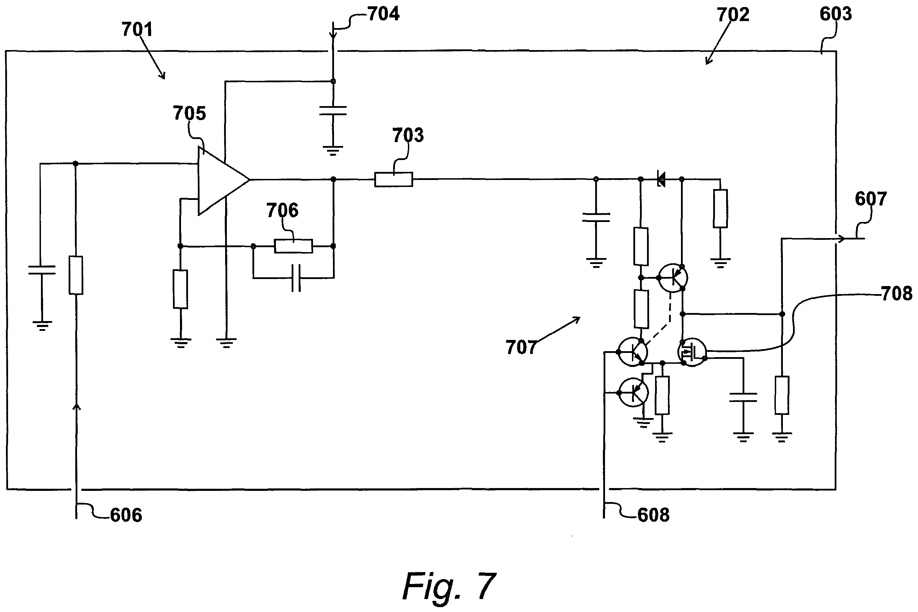

A schematic representation of the energising circuit **603** is shown in Figure 7. The energising circuit includes a voltage-control circuit **701** connected to a strobing circuit **702** via current-limiting resistor **703.** A voltage-input line **704** receives energising power from the power supply to energize an operational amplifier **705.** Amplifier **705** is configured as a comparator and receives a reference voltage via a feedback resistor **706.** This is compared against a voltage-control signal received on the voltage-control line **606**, to produce an input voltage for the strobing circuit **702.**

The strobing circuit includes two bipolar transistors configured as a Darlington pair **707**, in combination with a MOSFET (metal oxide silicon field effect transistor) **708.** This creates energising pulses with sharp rising edges, that are conveyed to the strobing line **607**, after receiving a trigger signal on line **608.**

### Figure 8

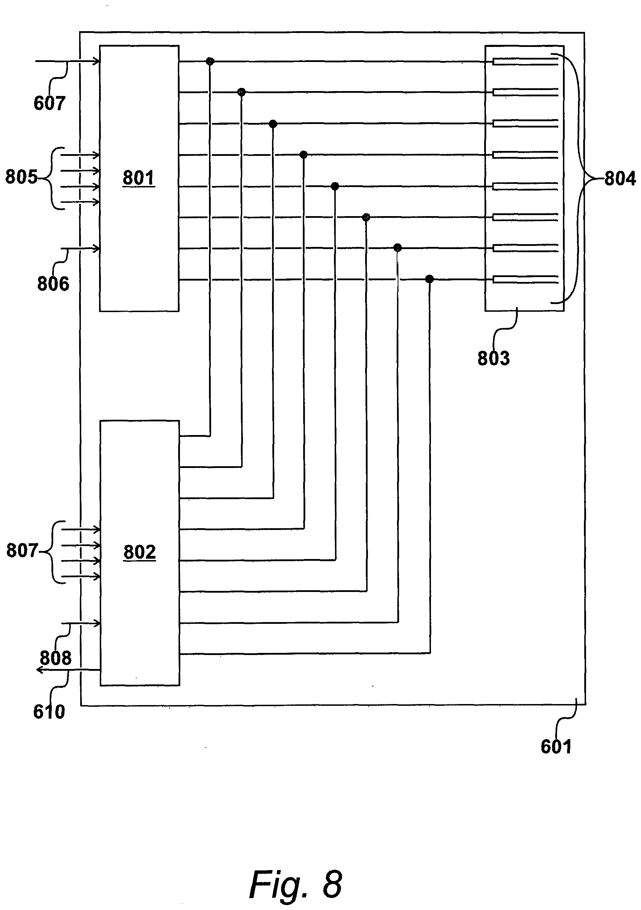

An example of a multiplexing environment is shown in Figure 8, in which a first multiplexing device **801** supplies energising input signals to a selected transmitter electrode, while a second multiplexing device **802** monitors output signals from a selected receiver electrode. A dielectric membrane **803** supports plural parallel electrodes coated with an insulating coating to allow them to be brought into contact with the subject's finger. Eight linear electrodes **804** are shown for illustrative purposes but more or fewer electrodes may be included.

An embodiment will be described in greater detail that includes two mutually offset groups, with fifteen electrodes in each group. Such an arrangement facilitates measurement in two dimensions; with the second group of electrodes being provided with respective multiplexing devices.

The first multiplexing device **601** includes first address lines **605** and an enabling line **606.** Similarly, the second multiplexing device **602** includes second address lines **607** and a second enabling line **608.** During each electrode coupling operation, addresses are supplied to the address busses but line selection does not occur until respective enabling signals have been received.

### Figure 9

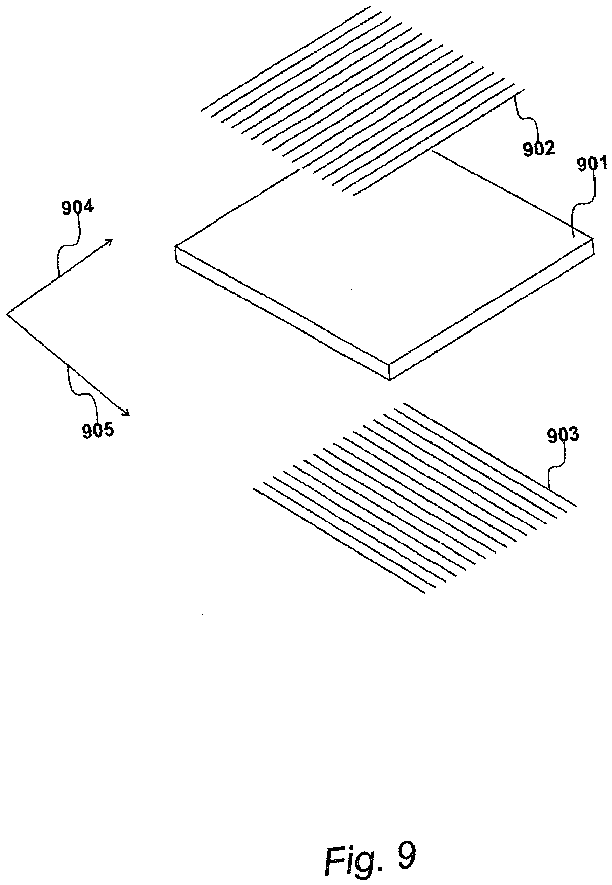

The provision of two mutually orthogonal electrode groups is illustrated in Figure 9. A dielectric membrane **901** is substantially similar to that shown in Figure 1. The subassembly includes a first group of electrodes **902**, along with a second group of electrodes **903.** In this example, fifteen electrodes are provided in each group and for each group, the electrodes are substantially linear and substantially parallel. However, the groups are mutually offset and, in this embodiment, arranged in mutually orthogonal orientations.

The first group of electrodes **901** are aligned in an x dimension, illustrated by a first arrow **904** and the second group of electrodes **903** are aligned in a y dimension, as illustrated by a second arrow **705.** Layering is achieved by coupling electrodes of a first set (selected from a group) and then repeating a scanning operation by coupling electrodes in a second set, selected from the same group. Thus, layering operations performed by the first group of electrodes **902** achieve a layering operation in the direction of the second arrow **905.** Similarly, the second group of electrodes **903** achieve a similar layering operation in the direction of the first arrow **904.**

### Figure 10

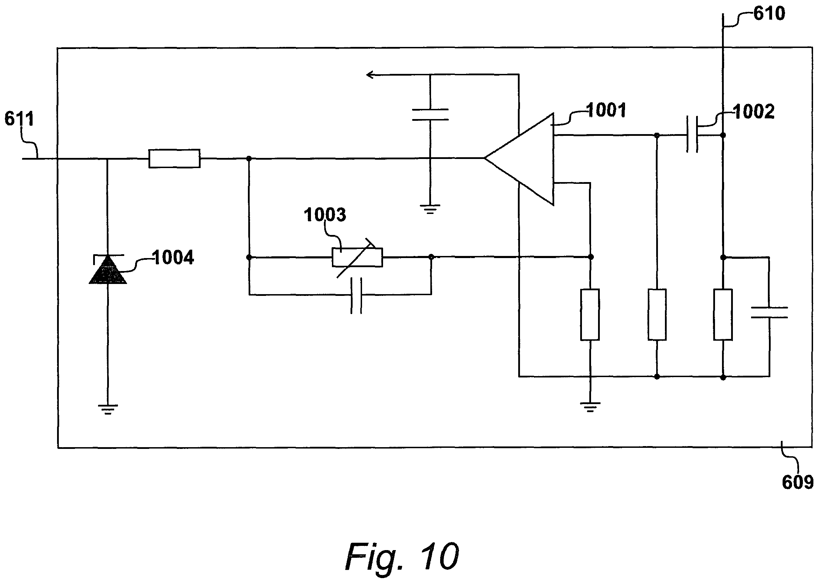

Analogue processing circuit **609** is shown in Figure 10. Signals received on the first analogue line **610** are supplied to a buffering amplifier **1001** via a decoupling capacitor **1002.** During an initial set-up procedure, a variable feedback resistor **1003** is trimmed to optimise the level of monitored signals supplied to the processor **602** via the second monitoring line **411.** A Zenner diode **1004** prevents excessive voltages being applied to the processor **402.**

### Figure 11

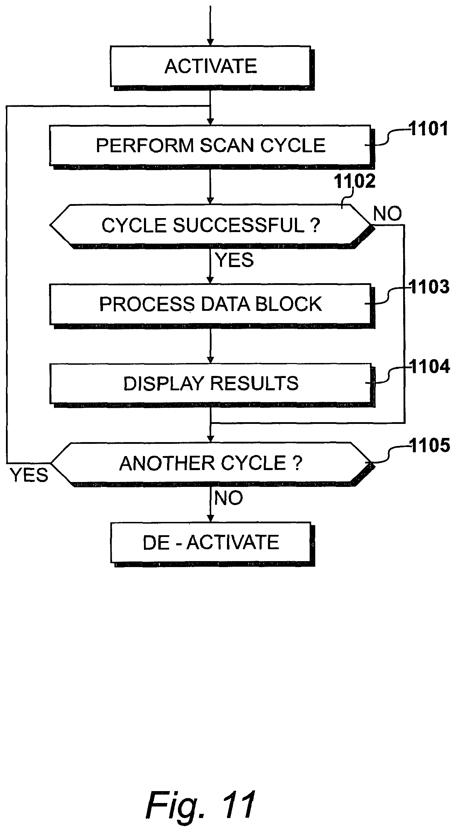

Procedures performed by processor **603** are illustrated in Figure 11. After activation of the apparatus, a scan cycle is performed at step **1101.** In an embodiment, this scan cycle consists of first performing calibration procedures, without human body contact, followed by testing procedures during which body contact has been established.

When body contact is made, a detector is configured to produce force data representing an applied force or pressure upon an active surface. The processor is configured to compare the force data against a first predetermined level. Thereafter, a decision is made as to whether it is possible to perform capacitive coupling procedure that capacitively couple selected pairs of the electrodes by producing electric fields that penetrate the tissue to produce monitored output signals, which occurs if the force data is higher then the predetermined level. Alternatively, if the force data is not higher than this predetermined level, these capacitive coupling procedures are inhibited.

Thus, as illustrated in Figure 11, after performing a scan cycle, a question is asked at step **1102** as to whether the cycle has been successful. Thus, if insufficient pressure was applied, the question asked at step **1102** will be answered in the negative. However, if a successful scanning cycle has been achieved, the question asked at step **1102** will be answered in the affirmative, allowing a data block to be processed at step **1103.** Thereafter, at step **1104,** results may be displayed.

### Figure 12

Thus, it has been appreciated that it is not possible to obtain satisfactory results if a subject does not apply their finger (or other body art) onto an application region with sufficient force. For satisfactory results to be obtained, a minimum allowable force is established by the first predetermined level. If the measured force does not achieve this level, further capacitive coupling procedures are inhibited.

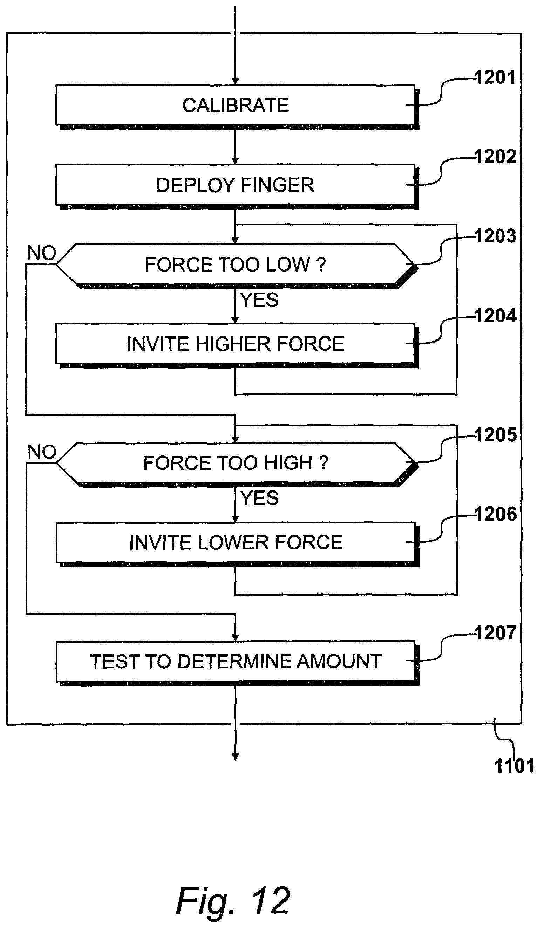

Further investigations also suggest that problems can arise if a subject applies too much force upon the active surface. Under these conditions, tissue and hence the distribution of blood within capillaries, is distorted, often to an extent where compensation cannot be achieved. Thus, in an embodiment, as illustrated in Figure 12, the processor is also configured to inhibit capacitive coupling procedures if the force data is above a second predetermined level.

An example of a scan cycle **1101** is illustrated in Figure 12. At step **1201** calibration procedures are conducted which, in an embodiment, replicate all of the procedures that will be performed during the testing stage. Thus, in the test data set, every data point has an equivalent point in the calibration data set.

After performing the calibration procedures, without a finger being in place, a subject is invited to deploy a finger at step **1202.** At step **1203** a question is asked as to whether the applied force is too low. Thus, at this stage, force data is compared against the first predetermined level. If this question is answered in the affirmative, to the effect that insufficient pressure has been applied, the subject is invited to apply higher force at step **1204.** Thus, in response to this invitation, the force is examined again at step **1203** and further invitations may be made if insufficient force continues to be applied.

In most situations, the question asked at step **1203** will eventually be answered in the negative, thereby allowing progress to step **1205.** However, as is known in the art, the loop created by steps **1203** and **1204** may include timeout provisions, resulting in the generation of an error message if a subject is unable to apply an appropriate level of force.

In response to the question asked at step **1203** being answered in the negative, confirming that a sufficient level of force has been applied, in this embodiment, a question is asked at step **1205** as to whether the applied force is too high. Thus, again, if this question is answered in the affirmative, the subject is invited to apply a lower force at step **1206.** Thus, again, the force may be tested at step **1205** and, under most circumstances, the question asked at step **1205** will eventually be answered in the negative such that testing procedures, similar to the calibration procedures, may be performed at step **1207.**

### Figure 13

The apparatus described herein evaluates an amount of a substance contained within blood circulating within human body tissue. Concentrations of many different substances may be evaluated, provided that they change the dielectric properties of the blood. These include inorganic, organic and biochemical substances. Features of the embodiment will be described with reference to an evaluation of glucose levels, given the prevalence of medical conditions requiring this substance to be evaluated regularly.

After performing a calibration procedure, the visual display unit **215** displays a message, along with a graphic, inviting a finger to be placed on the apparatus. Thus, instructions are displayed to a subject on the visual display unit, to assist the subject completing the overall evaluation procedure.

### Figure 14

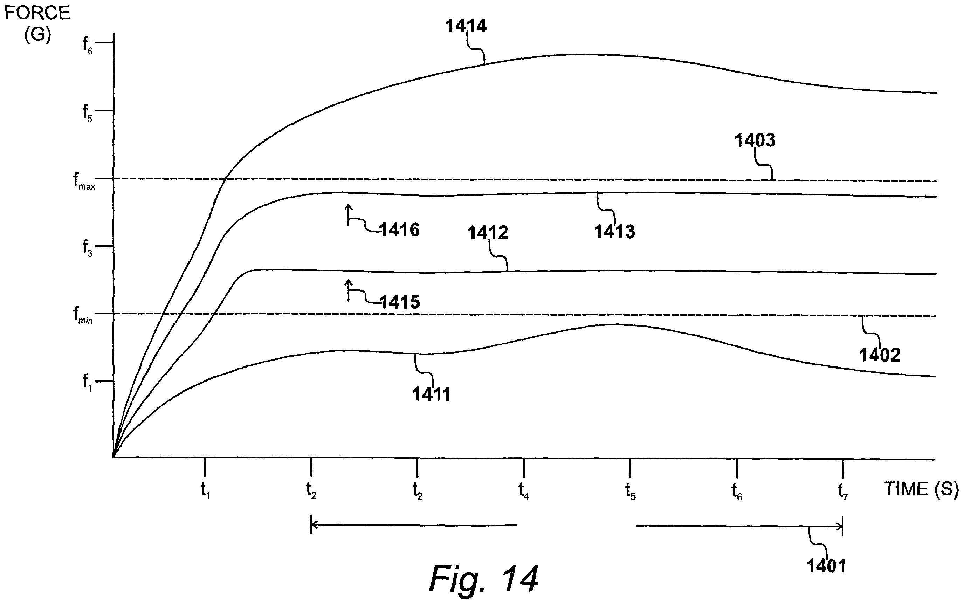

The present invention provides a method of evaluating an amount of a substance contained within blood circulating within the human body tissue. An application region of an apparatus is located to make skin contact at the position of the tissue, with the apparatus itself including electrodes arranged on a dielectric substrate to present an active surface at the application region. Force data is derived from a detector, as illustrated in Figure 14, where applied force is plotted against time. In an embodiment, a subject deploys a finger upon the application region of the apparatus. With a finger in place, evaluation takes place during an evaluation period **1401.**

Force data, representing applied force or applied pressure upon the apparatus, is compared against the first predetermined level **1402.** In an embodiment, a comparison is also made against a second predetermined level **1403.**

For the purposes of illustration, four deployments are illustrated in Figure **14**, consisting of a first deployment **1411**, a second deployment **1412**, a third deployment **1413** and a fourth deployment **1414.**

Referring to deployment **1411**, it can be seen that the level of force applied is below the first predetermined level **1402.** Thus, under these circumstances, the capacitive coupling procedures are inhibited and in accordance with an embodiment, the subject is invited to apply more pressure.

In the fourth deployment **1414**, the applied pressure is greater than the first predetermined level **1402** but, on this occasion, it is also greater than the second predetermined level **1403.** Thus, in this embodiment, the capacitive coupling procedures are again inhibited and a subject is invited to repeat the procedure while applying a lower pressure.

For the second deployment **1412** and the third deployment **1413**, the level of applied pressure is above the first predetermined level **1402** while being below the second predetermined level **1403.** Consequently, capacitive coupling procedures are performed that capacitively couple selected pairs of the electrodes by producing electric fields that penetrate the tissue to produce monitored output data.

Although the second deployment and the third deployment both allow test data to be produced, it should also be appreciated that different levels of force were maintained during period **1401.** Experiments have shown that a greater degree of accuracy can be achieved if account is made of this measured force. Thus, in an embodiment, before initiating the capacitive coupling procedures of the test, the applied pressure is actually measured and appropriate data recorded. Thus, for the second deployment **1412** force data is recorded, as indicated by arrow **1415.** Similarly, for the third deployment **1413**, force data is again recorded as indicated by arrow **1416.**

In an alternative embodiment, applied force is measured periodically during the scanning operation to ensure that this has not gone below the first predetermined level or above the second predetermined level. Under these circumstances, further scanning may again be inhibited and a subject invited to start the process again.

In a further embodiment, several pressure measurements may be recorded during the scanning process, even when each result continues to be between the lower threshold and the higher threshold. Thus, a first pressure value may be recorded at the start of layering using the first group of electrodes, with a new reading being taken at the start of layering with the second group of electrodes. In an embodiment, for each group, forward layering is followed by reverse layering and pressure values may be recorded separately for the forward and reverse components.

In an embodiment, temperature data and humidity are also measured and recorded. Temperature may be measured at the position of the finger, by detector **107** and inside the apparatus itself. Thus, all four measurements may be used to compensate measured values to improve overall accuracy. For glucose measurement, the aim is to obtain results that are at least as accurate, and preferably more accurate, than the results obtained using known invasive techniques.

Experiments have also shown that overall accuracy can be improved if the whole procedure is repeated, so that results can be averaged or compared. In an embodiment, three blocks or data are created for each subject by performing all of the procedures three times. If one of the data blocks produces results that are significantly different from the other two, this block is rejected and a selection is based on one of the other two or the other two are averaged.

### Figure 15

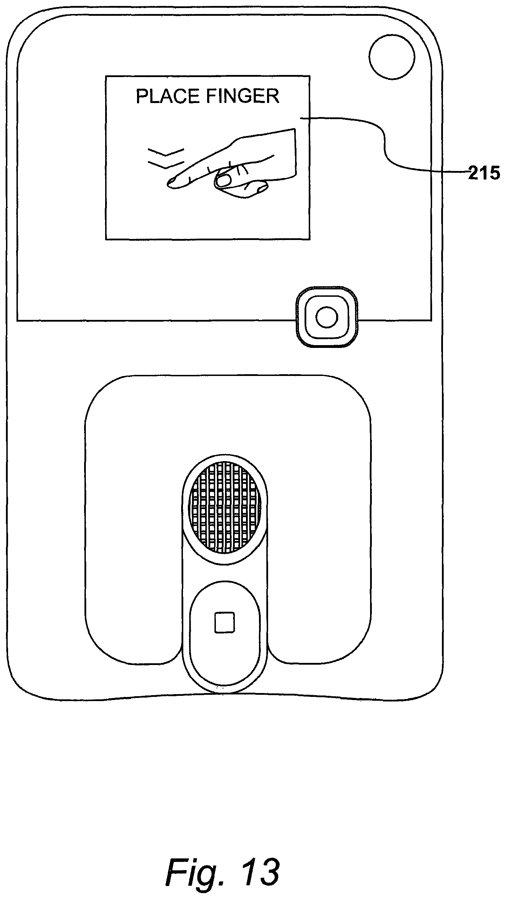

After having been invited to deploy a finger, as described with reference to Figure 13, a finger is engaged against the substantially parallel electrodes protected by the insulating coating. Thus, the electrodes are supported by the main circuit board and are exposed through the first membrane-exposing orifice in the housing.

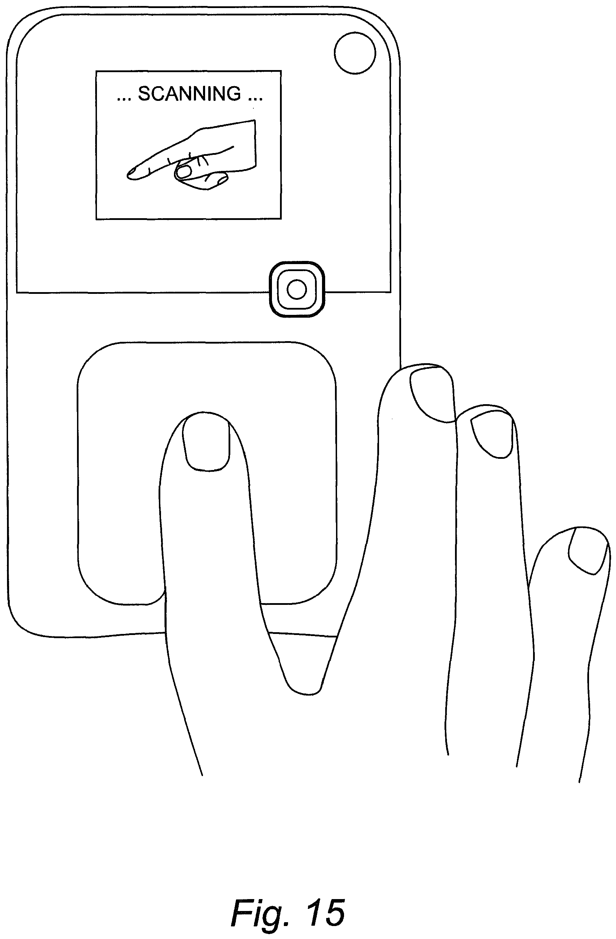

A control circuit energises and monitors selected electrodes to produce output data indicative of blood glucose concentrations. The visual display unit confirms this operation by identifying the apparatus as "scanning" as shown in Figure 15. Furthermore, throughout this procedure, the applied pressure is monitored by means of the force sensor supported by the bottom circuit board and force data is stored by the processor **602** when capacitive coupling procedures are being performed.

### Figure 16

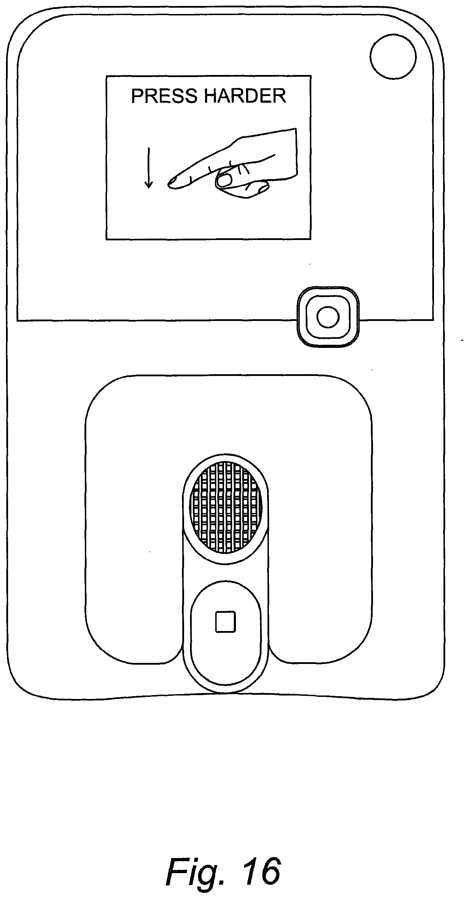

As previously described, step **1202** invites a subject to deploy a finger, as described with reference to Figure 13. An invitation of the type generated at step **1204** is illustrated in Figure 16. Thus, in response to an evaluation being made to the effect that applied force is too low, the subject, via the visual display **215**, is invited to press harder on the detector, so that the procedures may be repeated.

### Figure 17

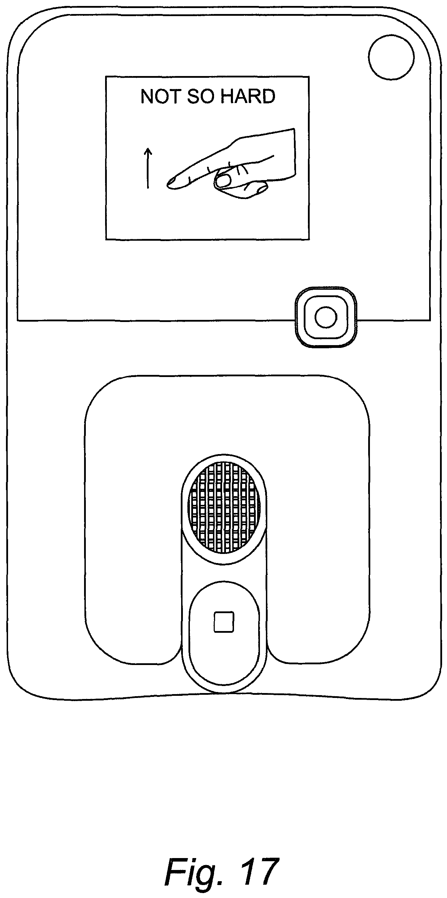

At step **1205**, a question has been asked as to whether the force was too high and when answered in the affirmative, step **1206** invites a user to deploy lower force. Thus, as illustrated in Figure 17, the visual display unit **215** invites the subject to press not so hard, such that this will hopefully result in the question asked at step **1205** being answered in the negative, such that test data may be established at step **1207.**

### Figure 18

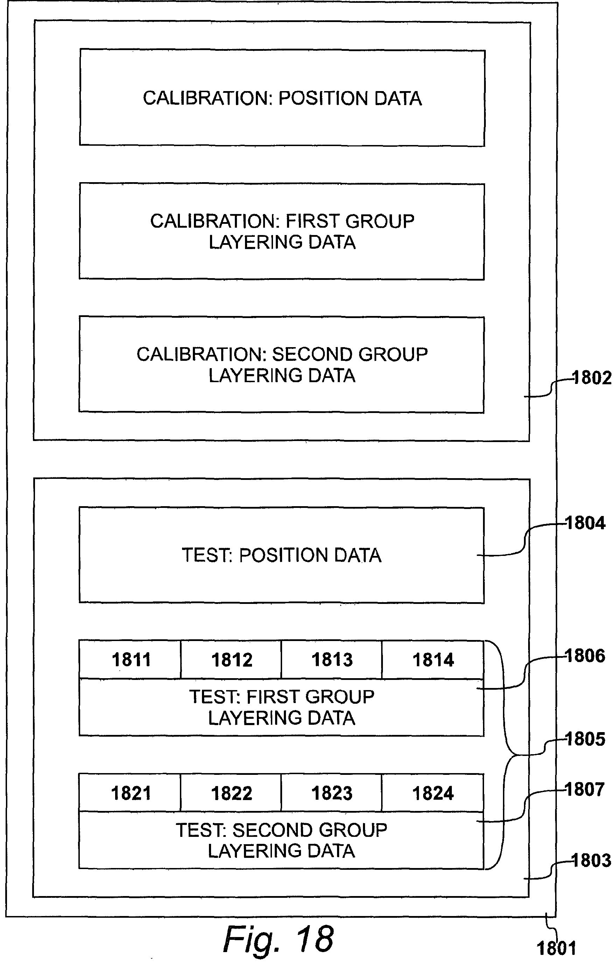

A scanning cycle, of the type usually performed for each individual subject, produces an output data block that is processed at step **1103.** An example of an output data block **1801** is shown in Figure 18. Each output data block consists of calibration data **1802** and test data **1803.** In this embodiment, the test data **1803** includes test position data **1804** and test layering data **1805.**

When two groups of electrodes are present, as described with reference to Figure 7, the test layering data **1805** comprises a first layering data set **1806** and a second layering data set **1807.** As illustrated in Figure 18, the calibration data also includes a similar data structure.

In this embodiment, the test layering data **1805** also includes the previously described environment data, consisting of force data, finger temperature data, internal device temperature data and humidity data. In this embodiment, respective measurements are recorded prior to first test layering procedures and prior to second test layering procedures. Thus, first group layering data **1806** includes force data **1811**, finger temperature data **1812**, internal temperature data **1813** and humidity data **1814.** Similarly, second group layering data **1807** includes force data **1821,** finger temperature data **1822,** internal temperature data **1823** and humidity data **1824.**

In an alternative embodiment, environment data is measured twice during each layering procedure. Firstly, at the start of forward layering and secondly at the start of reverse layering. Thus, four results are recorded for each data block. A scanning operation may produce a single data block or, in an embodiment, the procedures may be repeated twice to produce three data blocks, resulting in twelve environment data sets being recorded. Environment data may also be recorded during procedures other than layering during the overall scanning process.

### Figure 19

In an embodiment, processing step **1103** is performed using a machine learning system. In this embodiment, plural learning output data blocks are produced for a first selection of subjects, for which the extent to which a substance under investigation (such as glucose) is present, is known.

Plural learning output data blocks are deployed to prepare a machine learning system. Live output data blocks are then processed, at step **1103,** by means of the machine learning system, to produce respective evaluation data for the substance under investigation.

Machine learning systems of this type deploy regression algorithms to produce continuous outputs which, for example, may identify the level of glucose present within blood capillaries of the finger. In an alternative embodiment, a classification algorithm may be deployed to identify whether, for example, a glucose level is too low, normal or too high.

As is known in the art, each training example is represented by a vector and the training data is presented in a matrix. Through iterative operation of an objective function, supervised learning algorithms learn a function that can be used to predict the output associated with new inputs. Thus, an optimised function allows the algorithm to correctly determine the output for inputs that were not part of the original training data. Furthermore, after conducting a training procedure, the system will have learnt to perform the task required and it is therefore possible to provide an accurate evaluation of the amount of the substance (such as glucose) present in blood of the subject.

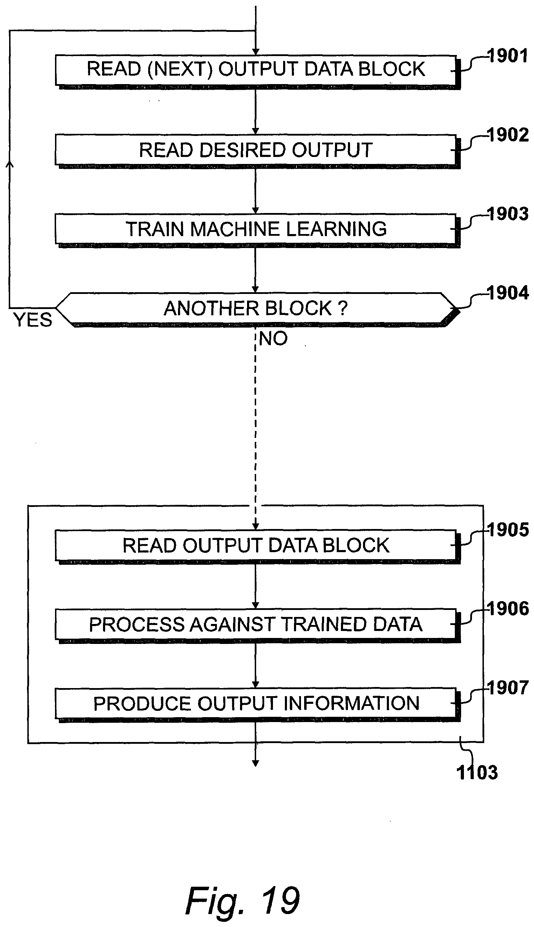

Operations performed with respect to the machine learning system are shown in Figure 19. Steps **1901** to **1904** relate to the training of the system, whereafter steps **1905** to **1908** relate to the deployment of the system at step **1103.**

At step **1901**, the next output data block, having a structure of the type described with reference to Figure 18, is read and the related desired output is then read at step **1902.** Thus, during the training operation, it is necessary to make measurements using alternative procedures. Thus, for example, when training a glucose monitoring apparatus, glucose measurements are made using conventional invasive techniques.

At step **1903**, the machine learning system is trained in response to the data received at step **1901** and step **1902**, whereafter at step **1904**, a question is asked as to whether another block is to be considered. When answered in the affirmative, the next output data block is read at step **1901.**

As is known in the art, the accuracy of the system will improve with the number of training iterations that are possible and this will be dependent upon the availability of data. During this process, random tests can be conducted to determine the accuracy of the system and a convergence towards accurate results should be witnessed. Only when an appropriate convergence has been achieved is it then possible to progress to the next stage.

Thus, the next stage represents procedures performed at step **1103.** Again, an output data block, of the type described with reference to Figure 18, is read at step **1905.** At step **1906**, this data block is processed against the trained data produced by the procedures described above. Thereafter, at step **1907**, output information is produced and from this, results are displayed at step **1104.**

### Figure 20

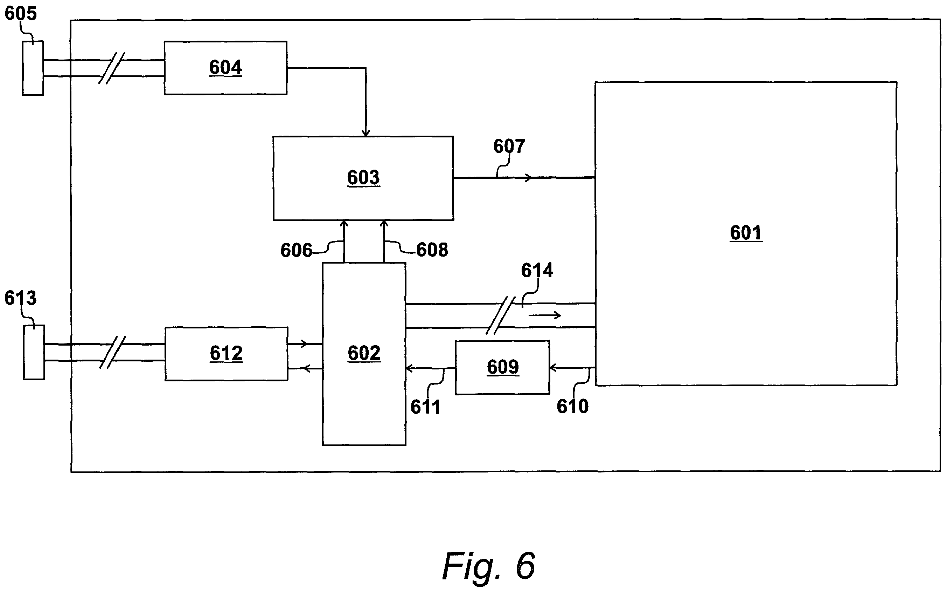

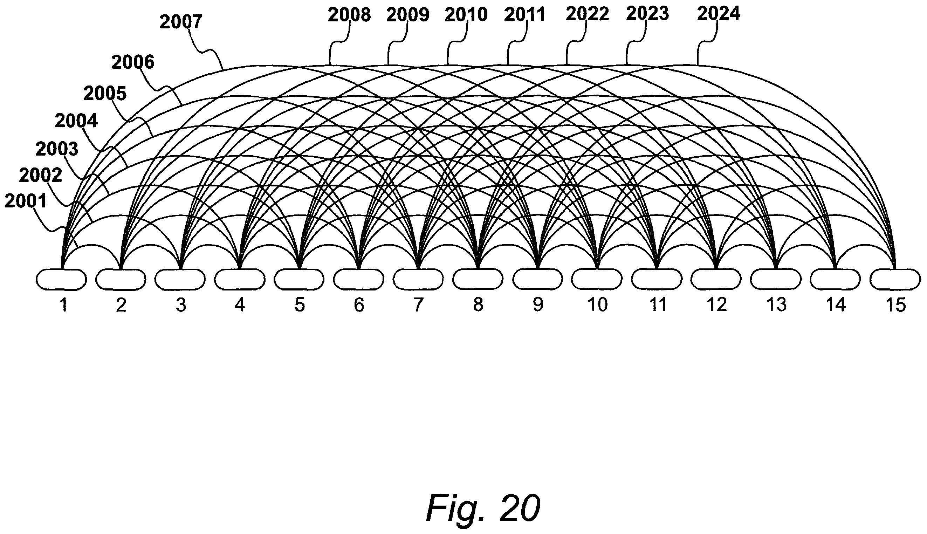

The electrodes of the first group **902** are shown in Figure 20, numbered **1** to **15.** Electronics within the evaluation apparatus, as described with reference to Figure 6, generates energisation pulses for application to any of the electrodes **1** to **15** as a transmitter electrode. In addition, output signals may be monitored from any remaining one of the electrodes as a receiver electrode, wherein a peak value of an output signal is indicative of permittivity and a decay rate of an output signal is indicative of conductivity. Thus, during each energisation operation, an energised transmitter electrode and a monitored receiver electrode define a capacitively coupled electrode pair.

From the available electrodes **1** to **15** of the selected group, a first set of n electrodes is selected. Thus, in the example shown in Figure 20, all fifteen electrodes are selected as the first set of n electrodes. However, it is not necessary for all of the available electrodes to be selected in this way, such that, in alternative embodiments, the selected set may contain fewer electrodes than the selected group.

Capacitively coupled electrode pairs are established, in which each of the first set of n electrodes is capacitively coupled with a second set of m electrodes selected from the first set of n electrodes. Thus, the second set of m electrodes is a subset of the first set of n electrodes. Thus, for each selected electrode of the first set, a respective second set of m electrodes is identified. These m electrodes are capacitively coupled with the selected electrode of the first set.

In an embodiment, this capacitive coupling may occur in parallel, requiring multiple analogue to digital conversion devices operating in parallel. However, in this embodiment, capacitive coupling occurs sequentially such that, at any instant, only one electrode of the first set is coupled with only one electrode of the second set. To achieve this coupling, either electrode may be energised as a transmitter, with the other electrode of the pair being monitored as the receiver.

Furthermore, in this embodiment, each second set of m electrodes are the nearest neighbouring electrodes to an electrode selected from the first set of n electrodes. Consequently, the number of electrodes present in the second set of m electrodes represents a degree of layering.

Following this method, all of the resulting electric fields are illustrated in Figure 20. The first set of n electrodes consists of all fifteen available electrodes within the group. The degree of layering is seven, as illustrated by electric fields **2001** to **2007.** Each of the first set of n electrodes is capacitively coupled with a second set of m electrodes. Thus, when considering the first electrode **1** as being a member of the first set, it is capacitively coupled with electrodes **2** to **8.** Electrode **1** is not capacitively coupled with electrodes **9** to **15.** Thus, the second set of m electrodes (**2** to **8**) with reference to the first electrode **1**, are the nearest neighbouring electrodes to electrode **1**, selected from the remaining electrodes of the first set. Thus, having achieved a seventh degree of layering, it would be necessary to couple electrode **1** with electrode **9**, should an eighth degree of layering be required.

The selected electrodes may be capacitively coupled in any order, provided that all of the electric fields illustrated in Figure 20 are realized.

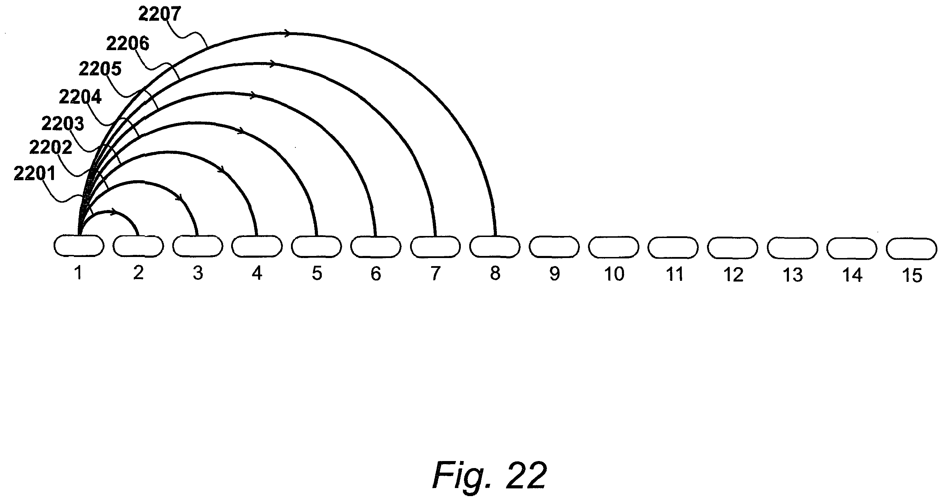

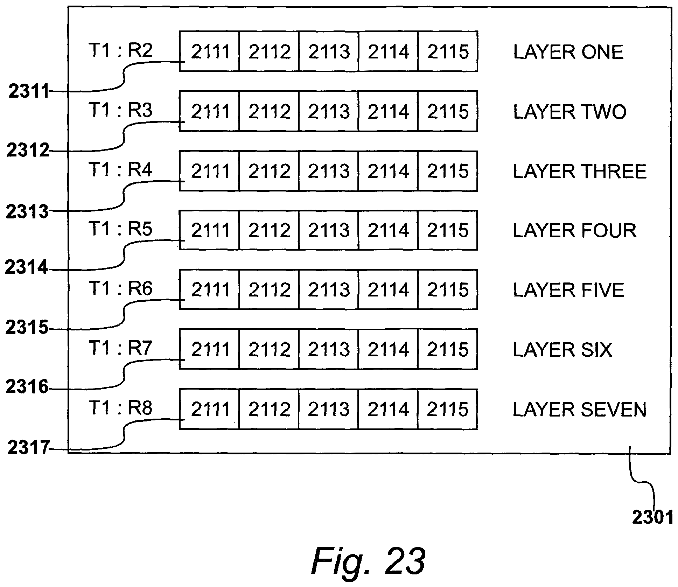

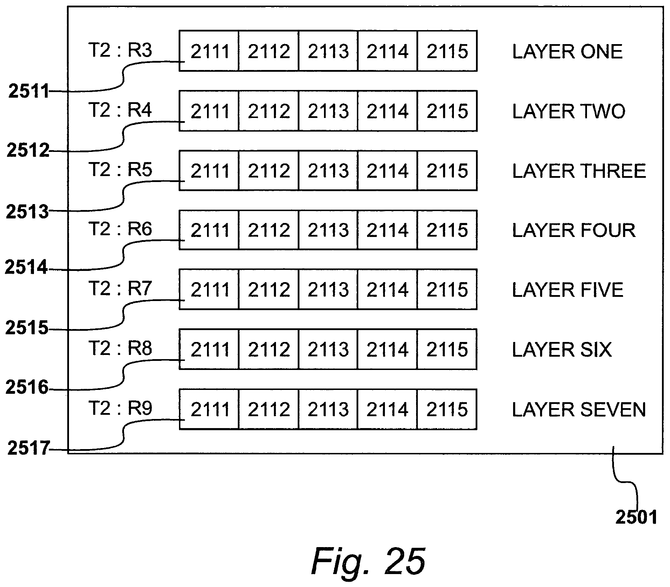

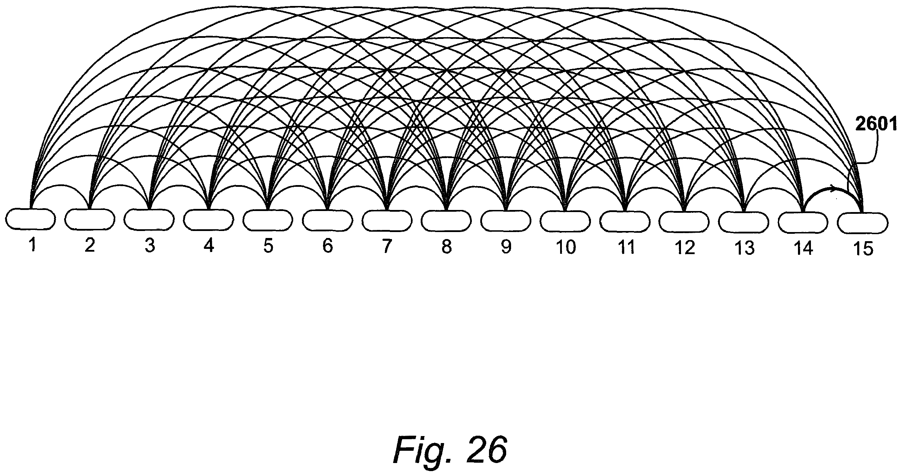

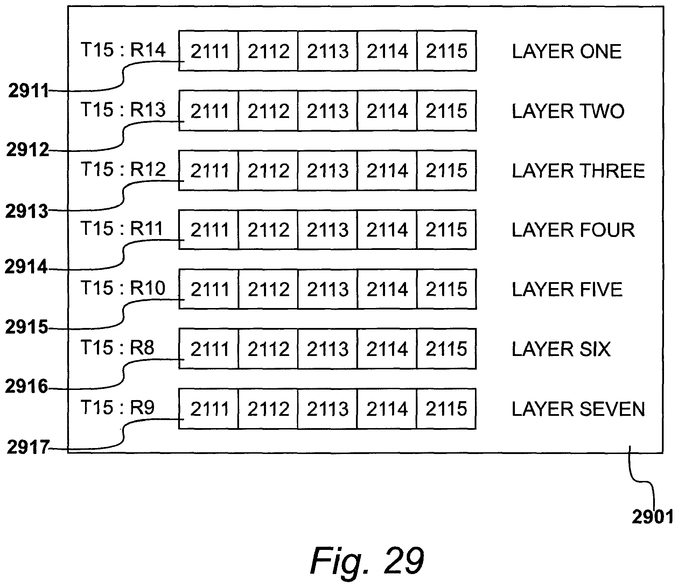

Previous investigations have identified advantages with respect to having multiple couplings with the end electrodes which, in this example, are identified as electrode **1** and electrode **15.** Such an approach provides useful layering data. However, in the present embodiment, many more layering opportunities are established through a dynamic process. The embodiments described herein develop these techniques to collect the required data in a systematic way and this approach will be described with reference to Figures 22 to 30. However, it should be appreciated that other approaches may be adopted to achieve the result of deriving data from coupling m electrodes that are the nearest neighbouring electrodes to an electrode selected from the first set of n electrodes.

As previously described, data derived from the seventh degree of layering is achieved by electric field **2007,** from the coupling of electrode **1** with electrode **8.** Similar seventh degree fields are shown at **2008** (coupling electrodes **2** and **9**), **2009** (coupling electrodes **3** and **10**), **2010** (coupling electrodes **4** and **11**), **2011** (coupling electrodes **5** and **12**), **2012** (coupling electrodes **6** and **13**), **2013** coupling electrodes **7** and **14**) and **2014** (coupling electrodes **8** and **15**).

### Figure 21

It can be appreciated that to achieve dynamic layering, a significant number of coupling operations are required for each scanning procedure, as described with reference to Figure 20. Furthermore, in an embodiment, each output signal produced from each capacitively coupled electrode pair is sampled to produce a coupling data set, in which a first sample of each coupling data set is indicative of permittivity and subsequent samples of each coupling data set are indicative of conductivity.

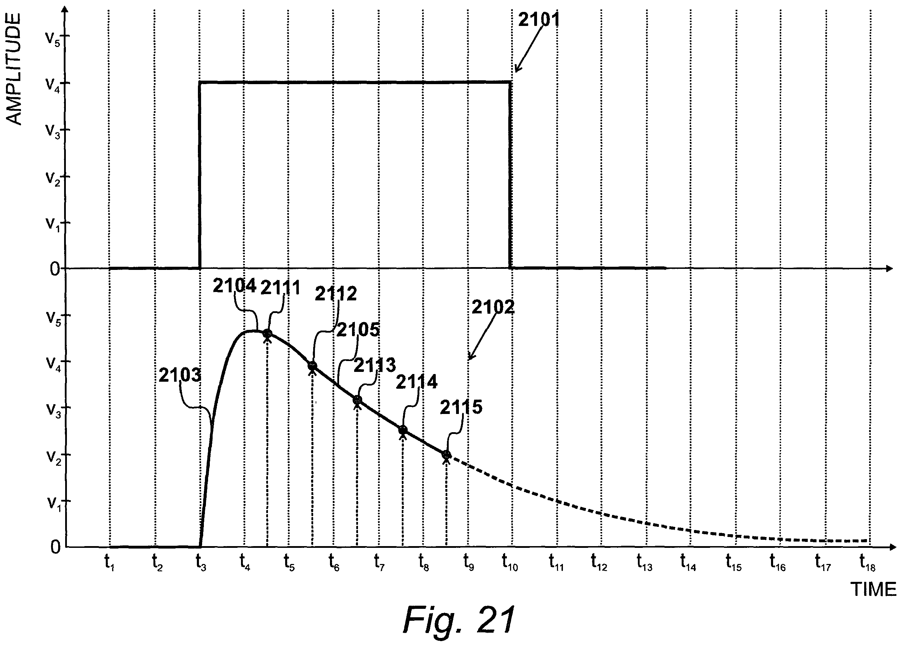

An energising input pulse **2101** is shown in Figure 21, plotted on a shared time axis with a monitored output signal **2102.** The energising input pulse **2101** rises relatively sharply to increase the high frequency components and hence produce a high-frequency-dependent response characteristic. Thus, in this embodiment, a frequency response is achieved without being required to sweep through many sinusoids of different frequencies.

The monitored output signal **2102** has been amplified by the circuit described with reference to Figure 10 and is then sampled by an analogue-to-digital convertor forming part of the processor **602.** The monitored output signal **2102** has a rising edge **2103**, a peak **2104** and a falling edge **2105.** The peak level **2104** will be determined predominantly by permittivity characteristics. Similarly, the falling edge **2105** represents a decay of the induced field and the rate of decay will be determined predominantly by conductivity characteristics. Thus, by recording multiple samples, it is possible to obtain rich coupling data sets. To achieve this, as illustrated in Figure 21, a first sampling point **2111** is followed by a second sampling point **2112**, followed by a third sampling point **2113**, a fourth sampling point **2114** and a fifth sampling point **2115.**

The processor **602** is responsible for initiating the energisation input signal, therefore the processor is instructed with an appropriate delay period before initiating the sampling process. This delay is determined empirically and aims to place the first sampling point at the peak value. However, a degree of tolerance is permitted, as illustrated in Figure 21, given that the same delay period is used for each coupling operation, allowing comparisons to be made between similar examples. However, for the purposes of this embodiment, it should be appreciated that each coupling operation results in the generation of an output signal substantially similar to that shown at **2102**, which in turn generates a coupling data set containing five data points. In other embodiments, more or fewer data samples may be recorded.

### Figure 22

The present embodiment is configured to capacitively couple electrodes to establish the pattern shown in Figure 20. The actual order for doing this is not relevant but the present embodiment takes a systematic approach to facilitate the programming of processor **602.** In particular, in an embodiment, capacitively coupled electrode pairs are established by sequentially selecting each of the n electrodes of the first set as an electrode in common. Furthermore, for each selected electrode in common, the procedure sequentially defines capacitively coupled electrode pairs with the second set of m nearest neighbouring electrodes. Thus, as illustrated in Figure 15, a procedure may start by selecting electrode **1** as the first electrode in common and then sequentially capacitively coupling electrode **1** with the m nearest neighbouring electrodes, which are electrodes **2** to **8**, when layering to the seventh degree is required.

To achieve the pattern shown in Figure 22, the electrode in common (electrode **1**) could be selected as a transmitter electrode or as a receiver electrode. In this embodiment, electrode **1** is selected as a transmitter electrode for each coupling operation, as represented by the arrow on each of the field lines. Thus, in an embodiment, the procedure is initiated by energising electrode **1** and monitoring electrode **2** to produce a first electric field **2201.** Electrode **1**, as the electrode in common, is energised again but with electrode **3** being monitored, to produce a second electric field **2202.** Again, as the electrode in common, electrode **1** is energised and a third electric field **2203** is produced by monitoring electrode **4.** This process is repeated with respect to electrode **5** being monitored, electrode **6** being monitored, electrode **7** being monitored and electrode **8** being monitored, producing respective electric fields **2204** to **2207.**

### Figure 23

The procedure described with reference to Figure 22 produces a common electrode data set **2301.** This is made up of seven coupling data sets **2311** to **2317**, from the first transmitter electrode **1** coupling with seven receiver electrodes **2** to **8.** In addition, as the distance between the electrodes increases, the degree of layering also increases. Thus, the first coupling data set **2311** may be identified as belonging to layer one, with the second belonging to layer two, the third belonging to layer three and so on, with the seventh **2317** belonging to layer seven.

As described with reference to Figure 21, each coupling data set consists of five data samples **2111** to **2115.**

### Figure 24

In this embodiment, the systematic selection of electrodes started by selecting a first end electrode, electrode **1**, as an electrode in common to produce the first common electrode data set **2301.** The process continues by sequentially selecting adjacent electrodes as electrodes in common in a first direction of (forward) dynamic layering, until a second end electrode (electrode **15** in this example) is reached.

Thus, in accordance with this embodiment, having selected electrode **1** as the electrode in common, adjacent electrode **2** is now selected as the electrode in common, resulting in the generation of electric fields **2401** to **2407.**

### Figure 25

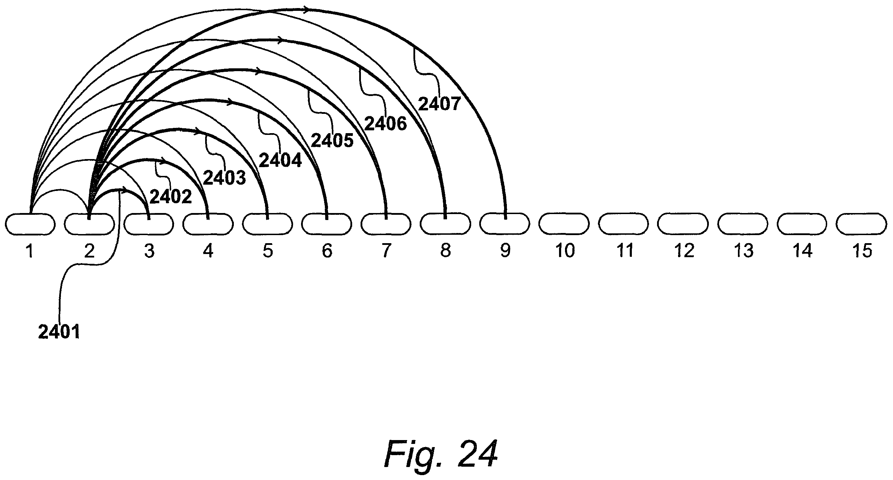

The coupling operations illustrated in Figure 24 result in the production of a second common electrode data set **2501.** Again, this produces a further seven coupling data sets **2511** to **2517**, representing the seven layers of penetration, each again including five coupling data sets **2101** to **2105.**

### Figure 26

The process of sequentially selecting adjacent electrodes as electrodes in common continues until a second end electrode is reached, as illustrated in Figure 19. When electrode **8** is selected as the electrode in common, a full seven layers of penetration can be achieved, giving the couplings that are possible with electrodes **9** to **15.** However, as subsequent electrodes are selected as the electrode in common, the level of penetration reduces until, as shown in Figure 19, when electrode **14** is selected as the electrode in common, it is only possible for it to couple with electrode **15**, as illustrated by electric field **2601.**

### Figure 27

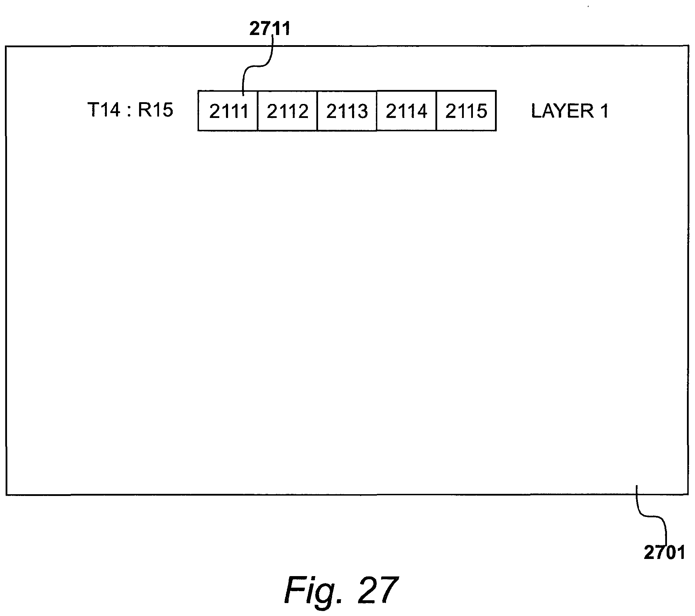

As illustrated in Figure 27, when electrode **14** is selected as the electrode in common, only one common electrode data set **2701** is produced. Again, this common electrode data set consists of five coupling data sets **2111** to **2115.**

### Figure 28

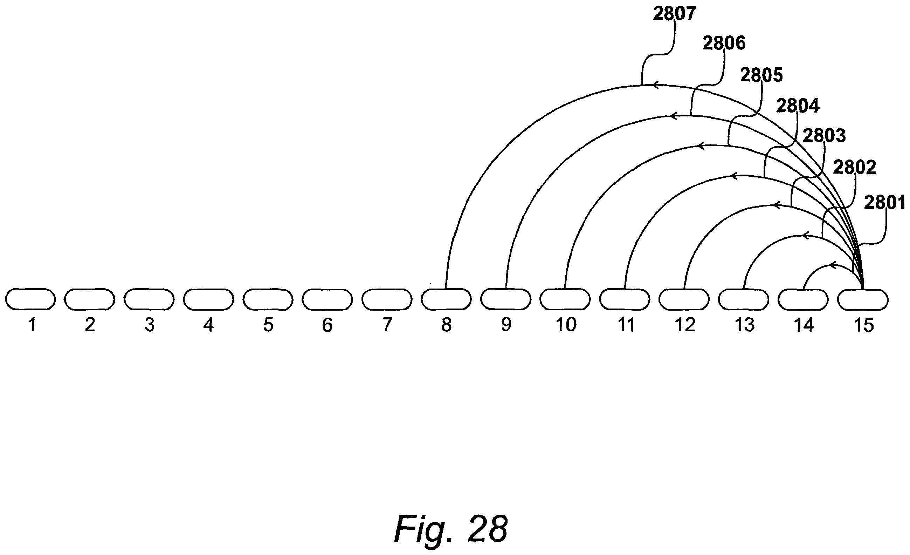

After reaching the second end electrode, as described with reference to Figure 19, the second end electrode **15** is now selected as an electrode in common, as shown in Figure 28. This results in the sequential generation of electric fields **2801** to **2807.**

Thereafter, adjacent electrodes, starting from electrode 14, are sequentially selected as the electrode in common, moving in the second direction of dynamic layering (reverse layering) until the first end (electrode **1**) is reached. Thus, for each selected electrode in common, such as electrode **15** in Figure 28, a second set of m electrodes are selected that are the nearest neighbours (**14** to **8**), but only in the direction of (reverse) dynamic layering.

### Figure 29

The procedures described with reference to Figure 28 produce a new common electrode data set **2201.** Again, on this occasion, penetration is possible to level seven, resulting in the generation of seven coupling data sets **2911** to **2917.**

### Figure 30

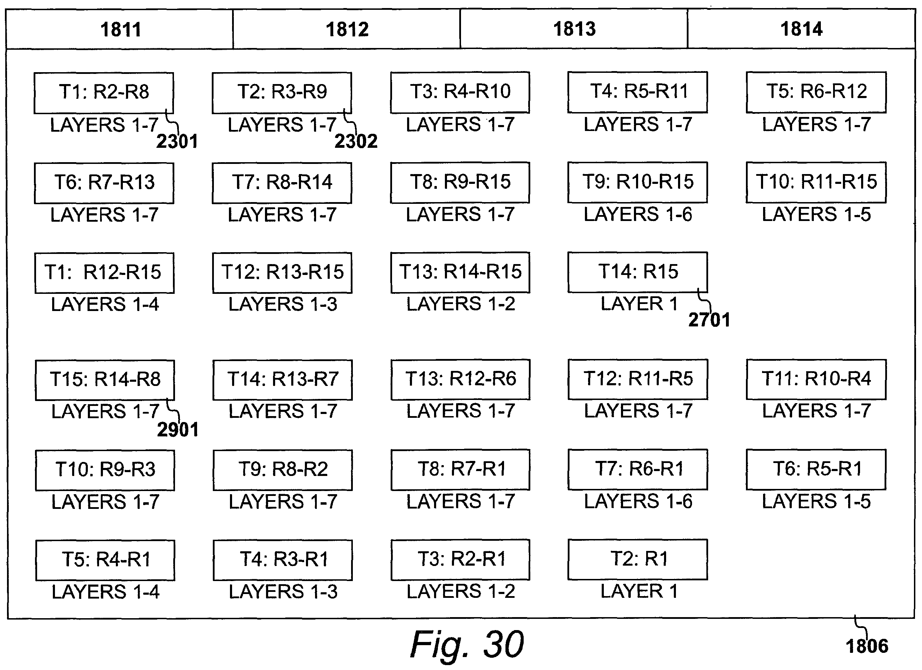

As illustrated in Figure 30, the procedure of forward dynamic layering followed by reverse dynamic layering produces twenty-eight common electrode data sets, which include common electrode data sets **2301**, **2302**, **2701** and **2201**, along with all the others making up a complete layering data set **2901.** Furthermore, in this embodiment, the procure also records force data **1811**, finger temperature data **1812**, internal temperature data **1813** and humidity data **1814.** In an alternative embodiment, this environment data is recorded separately for forward layering and reverse layering.

The result, as shown in Figure 30, is the production of the first electrode group layering data. For a complete scan, to produce the output data block **1001**, similar procedures are also performed for the calibration first group layering data, the calibration second group layering data and the test second group layering data **1007.**

### Figure 31

After the measuring process has completed, the visual display unit invites the subject to remove their finger, as illustrated in Figure 8. After removal, the method continues with the step of allowing the elastomeric material to expand, thereby returning the intermediate circuit board to its original position.

### Figure 32

After analysing output data received during the scanning procedure, it is possible for the visual display unit **215** to provide an indication of glucose concentration. Furthermore, in addition to providing a numerical value (representing four point one milli-moles of glucose in each litre of blood in this example), an indication may also be provided as to whether this concentration is considered to be low, normal or high. In an embodiment, for each of these possibilities, an appropriate colour is displayed. Thus, a low value may be presented in blue, a normal value in green and a high value in red.

It should be appreciated that similar graphical displays may be generated when evaluating concentrations of other blood containing substances.

## Claims

1. A method of determining an amount of a substance contained within blood circulating within human body tissue, **characterised by** the steps of:
locating an application region (105) of an apparatus to make skin contact at the position of said tissue, wherein said apparatus includes a plurality of substantially parallel electrodes arranged on a dielectric substrate to present an active surface (106) at said application region;
deriving force data from a detector (408);
comparing said force data, representing applied force/pressure of said skin contact upon said application region, against a first predetermined level (1402);
in response to said comparing step, either:
performing capacitive-coupling procedures that capacitively couple selected pairs of said electrodes by producing electric fields that penetrate said tissue to produce monitored output data 1803), if sufficient force has been applied, or
inhibiting said capacitive-coupling procedures if a sufficient force has not been applied, wherein
the amount of said substance is evaluated from said monitored output data when said monitored output data is produced;
wherein
said step of performing capacitive-coupling procedures comprises the steps of:
selecting a first set of n electrodes from said plurality of substantially parallel electrodes; and
for each of said electrodes in said first set:
selecting a second set of m electrodes from said plurality of substantially parallel electrodes, wherein the electrodes in said second set are the nearest neighbouring electrodes to said each electrode,
generating an energisation pulse for application to said each electrode as a transmitter electrode, and
monitoring output signals from each of said electrodes in said second set as a receiver electrode, wherein a peak value of an output signal is indicative of permittivity and a decay rate of an output signal is indicative of conductivity, such that during each energisation operation, an energised transmitter electrode and a monitored receiver electrode define a capacitively-coupled electrode pair;
such that a plurality of capacitively coupled electrode pairs are established, in which each electrode in said first set of electrodes is capacitively coupled with electrodes in a second set, and
the number m represents a degree of layering.

2. The method of claim **1**, further **characterised by** the step of indicating that an increase in applied force/pressure is required (Fig 16) if a sufficient force has not been applied.

3. The method of claim **1** or claim **2**, further **characterised by** the steps of:
comparing said force data against a second predetermined level (1403); and
inhibiting said capacitive coupling procedures if too much force has been applied.

4. The method of claim **3**, further **characterised by** the step of indicating that a reduction of applied force/pressure is required (Fig 17), if said force data is above said second predetermined threshold.

5. The method of any of claims **1** to **4**, further **characterised by** the step of storing force data when said capacitive coupling procedures are performed.

6. The method of claim **5**, **characterised in that** said step of performing capacitive-coupling procedures, comprises the steps of:
performing a plurality of procedure types; and
storing respective force data for each said procedure type.

7. The method of claim **6**, **characterised in that** said procedure types include calibration procedure types (1201) to produce calibration data (1802), in which capacitive-coupling procedures are performed prior to said step of making skin contact.

8. The method of claim **1**, **characterised in that** said step of establishing capacitively coupled electrode pairs comprises the steps of:
sequentially selecting each said n electrode of said first set as an electrode in common; and
for each said selected electrode in common, sequentially defining capacitively-coupled electrode pairs with a second set of m nearest neighbouring electrodes.

9. The method of any of claims **1** to **8**, further **characterised by** the steps of:
producing plural learning output data sets for a first group of subjects, for which the amount of a substance under investigation is known, based on permittivity and resistivity properties;
deploying said plural learning output data sets to prepare a machine-learning system; and
analysing live output data sets by means of said machine-learning system to produce respective amount data for said substance.

10. An apparatus for the non-invasive evaluation of an amount of a substance contained within blood circulating within human body tissue, **characterised by**:
an application region (105) arranged to make skin contact;
a plurality of substantially parallel electrodes (902) arranged on a dielectric substrate (901), thereby presenting an active surface (106) at the position of said application region;
a detector (408) configured to produce force data representing an applied force or pressure upon said active surface; and
a processor (603), wherein said processor is configured to:
compare said force data against a first predetermined level (1402);
perform capacitive-coupling procedures that capacitively couple selected pairs of said electrodes by producing electric fields that penetrate said tissue to produce monitored output data, if said force data is higher than said first predetermined level; and
inhibit said capacitive-coupling procedures if said force data is not above said first predetermined level;
wherein
said processor is configured to perform capacitive-coupling procedures by carrying out the steps of:
selecting a first set of n electrodes from said plurality of substantially parallel electrodes; and
for each of said electrodes in said first set:
selecting a second set of m electrodes from said plurality of substantially parallel electrodes, wherein the electrodes in said second set are the nearest neighbouring electrodes to said each electrode,
generating an energisation pulse for application to said each electrode as a transmitter electrode, and
monitoring output signals from each of said electrodes in said second set as a receiver electrode, wherein a peak value of an output signal is indicative of permittivity and a decay rate of an output signal is indicative of conductivity, such that during each energisation operation, an energised transmitter electrode and a monitored receiver electrode define a capacitively-coupled electrode pair;
such that a plurality of capacitively coupled electrode pairs are established, in which each electrode in said first set of electrodes is capacitively coupled with electrodes in a second set, and
the number m represents a degree of layering.

11. The apparatus of claim **10**, further **characterised by** a display device (215) configured to indicate that an increase in applied force/pressure is required if said capacitive coupling procedures have been inhibited.

12. The apparatus of claim **10** or claim **11**, further **characterised in that** said processor is also configured to inhibit said capacitive coupling procedures if said force data is above a second predetermined level (1403).

13. The apparatus of claim **12**, further **characterised by** including a display device (215) configured to indicate that a reduction in applied force/pressure is required.

14. The apparatus of any of claims **10** to **13**, wherein said processor is also configured to store force data (1811; 1821) when said capacitive coupling procedures are performed.

## Patentansprüche

1. Ein Verfahren zur Bestimmung der Menge einer Substanz, die im Blut enthalten ist, welches im menschlichen Körpergewebe zirkuliert, **gekennzeichnet durch** die folgenden Schritte:
Bestimmung eines Anwendungsbereichs (105) einer Vorrichtung, um an dieser Stelle des besagten Gewebes Hautkontakt herzustellen, wobei die besagte Vorrichtung eine Vielzahl im Wesentlichen paralleler Elektroden umfasst, die auf einem dielektrischen Substrat angeordnet sind, um für eine aktive Oberfläche (106) in dem besagten Anwendungsbereich zu sorgen;
Erlangen von Kraft-Messwerten mit einem Detektor (408);
Vergleichen der besagten Kraft-Messwerte, die die ausgeübte Kraft bzw. den Druck des besagten Hautkontakts auf den besagten Anwendungsbereich darstellen, mit einem ersten vorherbestimmten Wert (1402);
als Reaktion auf die besagte Vergleichsmaßnahme entweder:
Durchführen kapazitiver Kopplungsvorgänge, die ausgewählte Paare der besagten Elektroden kapazitiv koppeln, indem elektrische Felder erzeugt werden, die das Gewebe durchdringen, um überwachte Ausgabedaten (1803) zu erzeugen, sofern ausreichend Kraft angewendet wurde, oder
Unterbinden der besagten kapazitiven Kopplungsvorgänge, sofern keine ausreichende Kraft ausgeübt wurde, wobei
die Menge der besagten Substanz anhand der besagten überwachten Ausgabedaten bewertet wird, sobald die besagten überwachten Ausgabedaten erzeugt werden;
wobei die besagte Maßnahme des Durchführens kapazitiver Kopplungsvorgänge die folgenden Schritte umfasst:
Auswählen eines ersten Satzes von n-Elektroden aus der besagten Vielzahl im Wesentlichen paralleler Elektroden; und
für jede der besagten Elektroden des besagten ersten Satzes:
Auswählen eines zweiten Satzes von m-Elektroden aus der besagten Vielzahl im Wesentlichen paralleler Elektroden, wobei die Elektroden im besagten zweiten Satz die Elektroden sind, die jeder besagten Elektrode am nächsten stehen;
Erzeugen eines Erregungsimpulses zur Anwendung an jede besagte Elektrode als Senderelektrode, und
Überwachen von Ausgangssignalen von jeder der besagten Elektroden im besagten zweiten Satz als Empfängerelektrode, wobei ein Spitzenwert eines Ausgangssignals die Permittivität anzeigt und eine Abfallrate eines Ausgangssignals die Leitfähigkeit anzeigt, sodass während jedes Erregungsvorganges eine erregte Senderelektrode und eine überwachte Empfängerelektrode ein kapazitiv gekoppeltes Elektrodenpaar definieren;
sodass eine Vielzahl kapazitiv gekoppelter Elektrodenpaare gebildet werden, von denen jede Elektrode des besagten ersten Elektrodensatzes kapazitiv gekoppelt ist mit Elektroden eines zweiten Satzes, und
die Zahl m den Grad der Schichtung angibt.

2. Das Verfahren entsprechend Anspruch **1**, weiterhin **gekennzeichnet durch** den Schritt des Anzeigens, dass eine Erhöhung der ausgeübten Kraft bzw. des Drucks erforderlich ist (Abb. 16), sofern keine ausreichende Kraft ausgeübt wurde.

3. Das Verfahren entsprechend Anspruch **1** oder Anspruch **2**, weiterhin **gekennzeichnet durch** die folgenden Schritte:
Vergleichen der besagten Kraft-Messwerte mit einem zweiten vorherbestimmten Wert (1403); und
Unterbinden der besagten kapazitiven Kopplungsvorgänge, sofern zu viel Kraft ausgeübt wurde.

4. Das Verfahren entsprechend Anspruch **3**, weiterhin **gekennzeichnet durch** den Schritt des Anzeigens, dass eine Reduzierung der ausgeübten Kraft bzw. des Drucks erforderlich ist (Abb. 17), sofern die besagten Kraft-Messwerte über dem besagten zweiten vorherbestimmten Schwellenwert liegen.

5. Das Verfahren entsprechend jeglicher Ansprüche **1** bis **4**, weiterhin **gekennzeichnet durch** den Schritt des Speicherns von Kraft-Messwerten, sofern die besagten kapazitiven Kopplungsvorgänge durchgeführt werden.

6. Das Verfahren entsprechend Anspruch **5**, **dadurch gekennzeichnet, dass** der besagte Schritt des Durchführens kapazitiver Kopplungsvorgänge die folgenden Schritte umfasst:
Durchführen einer Vielzahl an Verfahrensarten; und
Speichern jeweiliger Kraft-Messwerte für jede besagte Verfahrensart.

7. Das Verfahren entsprechend Anspruch **6**, **dadurch gekennzeichnet, dass** die besagten Verfahrensarten Kalibrierungsverfahrensarten (1201) zur Erzeugung von Kalibrierungsmesswerten (1802) umfassen, bei denen kapazitive Kopplungsvorgänge vor dem besagten Schritt des Erwirkens von Hautkontakt durchgeführt werden.

8. Das Verfahren entsprechend Anspruch 1, **dadurch gekennzeichnet, dass** der besagte Schritt des Bildens kapazitiv gekoppelter Elektrodenpaare die folgenden Schritte umfasst:
sequenzielles Auswählen jeder der besagten n-Elektroden des besagten ersten Satzes als zugehörige Elektrode; und
für jede ausgewählte besagte zugehörige Elektrode das sequenzielle Definieren kapazitiv gekoppelter Elektrodenpaare mit einem zweiten Satz von m am nächsten positionierter Elektroden.

9. Das Verfahren entsprechend jeglicher Ansprüche **1** bis **8**, weiterhin **gekennzeichnet durch** die folgenden Schritte:
Erstellen mehrerer Lern-Ausgabedatensätze für eine erste Gruppe von Probanden, für die die Menge der zu untersuchenden Substanz bekannt ist, basierend auf Permittivitäts- und Widerstandseigenschaften;
Einsatz der besagten mehreren Lern-Ausgabendatensätze zur Erstellung eines Systems des maschinellen Lernens; und
Analysieren von Live-Ausgabedatensätzen mittels des besagten Systems des maschinellen Lernens, um entsprechende Mengendaten für die besagte Substanz zu erzeugen.

10. Eine Vorrichtung zur nicht-invasiven Bestimmung der Menge einer Substanz, die im Blut enthalten ist, welches im menschlichen Körpergewebe zirkuliert, **gekennzeichnet durch**:
einen Anwendungsbereich (105), der so gestaltet ist, dass er Hautkontakt herstellt;
eine Vielzahl im Wesentlichen paralleler Elektroden (902), die auf einem dielektrischen Substrat (901) angeordnet sind, und dadurch eine aktive Oberfläche (106) an der Position des besagten Anwendungsbereiches erzeugen;
ein Detektor (408), der so konfiguriert ist, dass er Kraft-Messwerte erzeugt, die eine auf die besagte aktive Oberfläche ausgeübte Kraft bzw. Druck darstellen; und
einen Prozessor (603), wobei der besagte Prozessor folgendermaßen konfiguriert ist:
Vergleichen der besagten Kraft-Messwerte mit einem ersten vorherbestimmten Wert (1402);
Durchführen von kapazitiven Kopplungsvorgängen, die ausgewählte Paare der besagten Elektroden kapazitiv koppeln, indem elektrische Felder erzeugt werden, die das besagte Gewebe durchdringen, um überwachte Ausgabedaten zu erzeugen, sofern die besagten Kraft-Messdaten höher als der besagte erste vorherbestimmte Wert sind; und
Unterbindung der besagten kapazitiven Kopplungsvorgänge, sofern die besagten Kraft-Messwerte nicht über dem besagten ersten vorherbestimmten Wert liegen;
wobei der besagte Prozessor so konfiguriert ist, dass er kapazitive Kopplungsvorgänge durchführt, indem er die folgenden Schritte ausführt:
Auswählen eines ersten Satzes von n-Elektroden aus der besagten Vielzahl im Wesentlichen paralleler Elektroden; und
für jede der besagten Elektroden des besagten ersten Satzes:
Auswählen eines zweiten Satzes von m-Elektroden aus der besagten Vielzahl im Wesentlichen paralleler Elektroden, wobei die Elektroden im besagten zweiten Satz die Elektroden sind, die jeder besagten Elektrode am nächsten stehen,
Erzeugen eines Erregungsimpulses zur Anwendung an jede besagte Elektrode als Senderelektrode, und
Überwachen von Ausgangssignalen von jeder der besagten Elektroden im besagten zweiten Satz als Empfängerelektrode, wobei ein Spitzenwert eines Ausgangssignals die Permittivität anzeigt und eine Abfallrate eines Ausgangssignals die Leitfähigkeit anzeigt, sodass während jedes Erregungsvorganges eine erregte Senderelektrode und eine überwachte Empfängerelektrode ein kapazitiv gekoppeltes Elektrodenpaar definieren;
sodass eine Vielzahl kapazitiv gekoppelter Elektrodenpaare gebildet werden, von denen jede Elektrode des besagten ersten Elektrodensatzes kapazitiv gekoppelt ist mit Elektroden eines zweiten Satzes, und
die Zahl m den Grad der Schichtung angibt.

11. Die Vorrichtung entsprechend Anspruch **10**, weiterhin **gekennzeichnet durch** ein Anzeigegerät (215), das so konfiguriert ist, dass es anzeigt, dass eine Erhöhung der ausgeübten Kraft bzw. des Drucks erforderlich ist, sofern die besagten kapazitiven Kopplungsvorgänge unterbunden wurden.

12. Die Vorrichtung entsprechend Anspruch **10** oder Anspruch **11**, weiterhin **dadurch gekennzeichnet, dass** der besagte Prozessor außerdem so konfiguriert ist, dass er die besagten kapazitiven Kopplungsvorgänge unterbindet, sofern die besagten Kraft-Messwerte über einem zweiten vorherbestimmten Wert (1403) liegen.

13. Die Vorrichtung entsprechend Anspruch **12**, weiterhin **dadurch gekennzeichnet, dass** sie ein Anzeigegerät (215) umfasst, das so konfiguriert ist, dass es anzeigt, dass eine Reduzierung der ausgeübten Kraft bzw. des Drucks erforderlich ist.

14. Die Vorrichtung entsprechend jeglicher Ansprüche **10** bis **13**, wobei der besagte Prozessor außerdem so konfiguriert ist, dass er Kraft-Messwerte (1811; 1821) speichert, sofern die kapazitiven Kopplungsvorgänge durchgeführt werden.

## Revendications

1. Un procédé de détermination d'une quantité d'une substance contenue dans le sang en circulation dans un tissu du corps humain, **caractérisé par** les étapes :
localisation d'une région d'application (105) d'un appareil pour établir un contact cutané au niveau dudit tissu, sachant que ledit appareil comprend une pluralité d'électrodes disposées sur un substrat diélectrique pour présenter une surface active (106) au niveau de ladite région d'application ;
dérivation des données relatives à la force provenant d'un détecteur (408) ;
comparaison desdites données relatives à la force, représentant la force/pression appliquée par ledit contact cutané sur ladite région d'application, par rapport à un premier niveau prédéterminé (1402) ;
réalisation de procédures de couplage capacitif couplant de manière capacitive des paires sélectionnées desdites électrodes en produisant des champs électriques qui pénètrent ledit tissu pour produire des données de sortie surveillées (1803), si une force suffisante a été appliquée, ou
inhibition desdites procédures de couplage capacitif, si une force suffisante n'a pas été appliquée, sachant que
la quantité de ladite substance est évaluée à partir desdites données de sortie surveillées lors de la production desdites données de sortie surveillées ;
sachant que ladite étape de réalisation des procédures de couplage capacitif comprend les étapes suivantes :
sélection d'un premier ensemble de n électrodes parmi ladite pluralité d'électrodes sensiblement parallèles ; et
pour chacune desdites électrodes dans ledit premier ensemble :
sélection d'un second ensemble de m électrodes parmi ladite pluralité d'électrodes sensiblement parallèles, sachant que les électrodes dans ledit second ensemble correspondent aux électrodes voisines les plus proches de chacune desdites électrodes,
génération d'une impulsion d'excitation destinée à être appliquée à chacune desdites électrodes en tant qu'électrode émettrice, et
surveillance des signaux de sortie provenant de chacune desdites électrodes dans ledit second ensemble en tant qu'électrode réceptrice, sachant qu'une valeur de crête d'un signal de sortie indique la permittivité et un taux de décroissance d'un signal de sortie indique la conductivité, de telle sorte qu'au cours de chaque opération d'excitation, une électrode émettrice excitée et une électrode réceptrice surveillée constituent une paire d'électrodes couplées de manière capacitive ;
de telle sorte qu'une pluralité de paires d'électrodes couplées de manière capacitive soit établie, dans laquelle chaque électrode dans ledit premier ensemble d'électrodes est couplée de manière capacitive avec des électrodes dans un second ensemble, et
le nombre m représente un degré de stratification.

2. Le procédé selon la revendication **1**, **caractérisé en outre par** l'étape consistant à indiquer qu'une augmentation de la force/pression appliquée est nécessaire (III. 16) si une force suffisante n'a pas été appliquée.

3. Le procédé selon la revendication **1** ou la revendication **2**, **caractérisé en outre par** les étapes suivantes :
comparaison desdites données relatives à la force par rapport à un second niveau prédéterminé (1403) ; et
inhibition desdites procédures de couplage capacitif si une force trop importante a été appliquée.

4. Le procédé selon la revendication **3**, **caractérisé en outre par** l'étape consistant à indiquer qu'une réduction de la force/pression appliquée est nécessaire (III. 17), si lesdites données relatives à la force est supérieure audit second seuil prédéterminé.

5. Le procédé selon les revendications **1** à **4**, **caractérisé en outre par** l'étape consistant à conserver des données relatives à la force lors de la réalisation desdites procédures de couplage capacitif.

6. Le procédé selon la revendication **5**, **caractérisé en ce que** ladite étape consistant à réaliser des procédures de couplage capacitif, comporte les étapes suivantes :
réalisation d'une pluralité de types de procédures ; et
conservation des données relatives à la force spécifiques pour chacun desdits types de procédures.

7. Le procédé selon la revendication **6**, **caractérisé en ce que** lesdits types de procédures comprennent des types de procédures d'étalonnage (1201) pour produire des données d'étalonnage (1802), dans lesquelles des procédures de couplage capacitif sont réalisées avant ladite étape consistant à établir un contact cutané.

8. Le procédé selon la revendication **1**, **caractérisé en ce que** ladite étape consistant à établir des paires d'électrodes couplées de manière capacitive comporte les étapes suivantes :
sélection séquentielle de chacune desdites n électrodes dudit premier ensemble en tant qu'électrode commune ; et
pour chacune desdites électrodes communes sélectionnées, définition séquentielle de paires d'électrodes couplées de manière capacitive avec un second ensemble de m électrodes voisines les plus proches.

9. Le procédé selon l'une quelconque des revendications **1** à **8**, **caractérisé en outre par** les étapes suivantes :
production de multiples ensembles de données de sortie d'apprentissage pour un premier groupe de sujets, pour lequel la quantité d'une substance à l'étude est connue, sur la base de propriété de permittivité et de résistivité ;
déploiement desdits multiples ensembles de données de sortie d'apprentissage pour préparer un système d'apprentissage automatique ; et
analyse des ensembles de données de sortie en direct au moyen dudit système d'apprentissage automatique pour produire des données de quantité spécifiques pour ladite substance.

10. Un appareil destiné à évaluer une quantité d'une substance contenue dans le sang en circulation dans un tissu du corps humain, **caractérisé par** :
une région d'application (105) disposée pour établir un contact cutané ;
une pluralité d'électrodes sensiblement parallèles (902) disposées sur un substrat diélectrique (901), présentant ainsi une surface active (106) au niveau de ladite région d'application ;
un détecteur (408) configuré pour produire des données relatives à la force représentant une force ou une pression appliquée sur ladite surface active ; et
un processeur (603), sachant que ledit processeur est configuré pour :
comparer lesdites données relatives à la force par rapport à un premier niveau prédéterminé (1402) ;
réaliser des procédures de couplage capacitif couplant des paires sélectionnées desdites électrodes de manière capacitive en produisant des champs électriques qui pénètrent ledit tissu pour produire des données de sortie surveillées, si lesdites données relatives à la force sont supérieures audit premier niveau prédéterminé ; et
inhiber lesdites procédures de couplage capacitive si lesdites données relatives à la force ne sont pas supérieures audit premier niveau prédéterminé ;
sachant que ledit processeur est configuré pour réaliser des procédures de couplage capacitive en effectuant les étapes suivantes :
sélection d'un premier ensemble de n électrodes à partir de ladite pluralité d'électrodes sensiblement parallèles ; et
pour chacune desdites électrodes dans ledit premier ensemble :
sélection d'un premier ensemble de n électrodes parmi ladite pluralité d'électrodes sensiblement parallèles, sachant que les électrodes dans ledit second ensemble correspondent aux électrodes voisines les plus proches de chacune desdites électrodes,
génération d'une impulsion d'excitation destinée à être appliquée à chacune desdites électrodes en tant qu'électrode émettrice, et
surveillance des signaux de sortie provenant de chacune desdites électrodes dans ledit second ensemble en tant qu'électrode réceptrice, sachant qu'une valeur de crête d'un signal de sortie indique la permittivité et un taux de décroissance d'un signal de sortie indique la conductivité, de telle sorte qu'au cours de chaque opération d'excitation, une électrode émettrice excitée et une électrode réceptrice surveillée constituent une paire d'électrodes couplées de manière capacitive ;
de telle sorte qu'au cours de chaque opération d'excitation, une électrode émettrice excitée et une électrode réceptrice surveillée constituent une paire d'électrodes couplées de manière capacitive ; de telle sorte qu'une pluralité de paires d'électrodes couplées de manière capacitive soit établie, dans laquelle chaque électrode dans ledit premier ensemble d'électrodes est couplée de manière capacitive avec des électrodes dans un second ensemble, et le nombre m représente un degré de stratification.

11. L'appareil selon la revendication **10**, **caractérisé en outre par** un dispositif d'affichage (215) configuré pour indiquer qu'une augmentation de la force/pression appliquée est nécessaire si les procédures de couplage capacitif ont été inhibées.

12. L'appareil selon la revendication **10** ou la revendication **11**, **caractérisé en outre en ce que** ledit processeur est également configuré pour inhiber les procédures de couplage capacitif si les données relatives à la force sont supérieures à un second niveau prédéterminé (1403).

13. L'appareil selon la revendication **12**, **caractérisé en outre en ce qu'**il comprend un dispositif d'affichage (215) configuré pour indiquer qu'une réduction de la force/pression appliquée est nécessaire.

14. L'appareil selon l'une quelconque des revendications **10** à **13**, sachant que ledit processeur est également configuré pour stocker des données relatives à la force (1811 ; 1821) lors de la réalisation desdites procédures de couplage capacitif.
